# FASCICULE DE BREVET EUROPEEN

(11) **EP 2 678 691 B1**
(45) Date de publication et mention de la délivrance du brevet: **13.07.2016**
(21) Numéro de dépôt: 12711924.6
(22) Date de dépôt: 23.02.2012
(51) Int. Cl.: G01N 33/84

(54) **DOSAGE D'ARSENIC PAR ELISA**
ELISA ZUM NACHWEIS VON ARSEN
ELISA FOR DETECTING ARSENIC

(30) Priorité: 25.02.2011 FR 1151566
(43) Date de publication de la demande: 01.01.2014
(73) Titulaire: Université de Lorraine, 54052 Nancy Cedex (FR)
(72) Inventeur: POUPIN, Pascal, F-57070 Metz (FR)
(74) Mandataire: Novagraaf Technologies
(86) Numéro de dépôt international: PCT/FR2012/050377
(87) Numéro de publication internationale: WO 2012/114046

(56) Documents cités:
- WO-A1-2010/004736
- US-A- 5 459 040
- US-A1- 2003 096 275
- KAWAKAMI Y. ET AL.: "Application of fluorescent protein-tagged trans factors and immobilized cis elements to monitoring of toxic metals based on in vitro protein-DNA interactions", BIOSENS. BIOELECTR., vol. 26, no. 4, 15 décembre 2010 (2010-12-15), pages 1466-1473, XP027546866,
- STOCKER J. ET AL.: "Development of a set of simple bacterial biosensors for quantitative and rapid measurements of arsenite and arsenate in potable water", ENVIRON. SCI. TECHNOL., vol. 37, no. 20, 15 octobre 2003 (2003-10-15), pages 4743-4750, XP002449112,
- JINBO XIONG ET AL.: 'Arsenic resistant bacteria in mining wastes from Shangrao coal mine of China' ENV. SCI. TECHNOL. vol. I, 2006, pages 535 - 540, XP055251105

## Description

### Domaine technique

La présente invention se rapporte à un procédé d'analyse d'arsenic dans un échantillon, notamment dans de l'eau, comprenant une étape de détection par une technique ELISA. Elle se rapporte également à un kit destiné à la détection d'arsenic dans un échantillon.

La présente invention trouve des applications notamment dans les domaines de la santé et de la sécurité alimentaire.

Dans la description ci-dessous, les références entre crochets (**[ ]**) renvoient à la liste des références présentée à la fin des exemples.

### Etat de la technique

L'arsenic (As) est un élément chimique largement répandu dans la nature. Il est présent dans l'atmosphère, les sols, les sédiments, les roches, les eaux de surface et les nappes phréatiques. L'arsenic est également présent dans le sous-sol. Il est le vingtième élément le plus abondant de la croûte terrestre. Ce composé est par ailleurs un constituant majeur de plus de deux cents minéraux, dont les plus courants sont l'arsénopyrite (FeAsS), le réalgar (AsS), ou l'orpiment (As₂S₃).

Parmi les métalloïdes et les métaux lourds, l'arsenic est l'un des rares éléments qui peut être mobilisé aux valeurs de pH caractéristiques des eaux souterraines, c'est-à-dire entre pH 6,5 et pH 8,5. Les espèces inorganiques, essentiellement la forme trivalente arsénite (As[III]) et la forme pentavalente arséniate (As[V]), sont prédominantes. Les formes organiques résultant de l'activité biologique peuvent se rencontrer occasionnellement, mais principalement dans les eaux de surface, et ne sont pas quantitativement importantes. Les concentrations en arsenic dans l'eau sont variables, dues principalement au contexte géologique et aux caractéristiques chimiques du milieu considéré. Les plus fortes concentrations se retrouvent dans les eaux souterraines, environnement où les conditions physico-chimiques favorisent la mobilisation de l'arsenic. La contamination de l'eau par l'arsenic est généralement naturelle mais elle peut aussi être d'origine anthropique.

Les apports d'arsenic dans l'atmosphère sont dus à l'érosion éolienne des sols, les émissions volcaniques, les embruns marins, les feux de forêts et à l'activité industrielle. Les plus importantes sources anthropiques sont dues à la fonte des minerais et la combustion des énergies fossiles riches en arsenic qui produisent de l'oxyde d'arsenic (As₂O₃) très toxique, et dont le cuivre pour la fonte et charbon pour la combustion représentent 60% des émissions. Le temps de résidence de l'arsenic dans l'atmosphère est estimé à moins de 10 jours avant sa retombée sous forme de poussière, ou avec les pluies. Une concentration atmosphérique plus importante en arsenic est observée près de sites de production de fonte des minerais et de combustion des énergies fossiles riches en arsenic (Bisson M., Houeix N., Hulot C., Lacroix G., Lefevre J.P., Leveque S., Magaud H., Morin A. 2006. Arsenic et ses dérivés inorganiques **[1]**).

L'ingestion d'arsenic peut conduire, chez l'homme, à une intoxication aiguë ou chronique. L'intoxication aiguë est due à l'ingestion d'une quantité importante d'arsenic (1 à 2 mg/kg/j).

Les effets aigus de l'intoxication à l'arsenic inorganique surviennent dans les dix minutes à quelques heures suivant l'ingestion. Ils sont typiquement gastro-intestinaux avec des nausées, des vomissements, des douleurs abdominales et des diarrhées. Ces symptômes s'accompagnent souvent d'une diminution de la pression artérielle, de convulsions, d'un coma associé à une détresse respiratoire, un oedème pulmonaire, une anémie hémolytique, et une insuffisance rénale qui peuvent provoquer la mort.

L'intoxication chronique à l'arsenic est due à une ingestion de faibles doses sur une longue période, c'est-à-dire plusieurs années. La principale cause de cette forme d'intoxication est la consommation d'une eau de boisson contaminée par de l'arsenic. L'exposition prolongée à l'arsenic est à l'origine de cancers de la peau, des poumons, de la vessie, et des reins ainsi que des changements cutanés tels que la modification de pigmentation de la peau et/ou des hyperkératoses. Ces intoxications peuvent aussi avoir des effets cardiovasculaires, des atteintes vasculaires périphériques telles que les gangrènes, des effets hématologiques telle qu'une anémie ou une leucopénie, des atteintes du système nerveux et des effets hépatiques (**[1]** et Organisation Mondiale de la Santé. 2006. Réduction des concentrations d'arsenic et sécurité de la nappe phréatique. EB118/14 **[2]**).

En outre l'arsenic inorganique (Asi) a été classé cancérigène pour l'homme par le Centre International de Recherche sur le Cancer (CIRC) et par l'Agence de Protection de l'Environnement américaine (US EPA).

Ainsi, dès 1958, l'organisation mondiale de la santé (OMS) a pris une position officielle sur les risques sanitaires liés à la présence d'arsenic dans l'eau de boisson **[2].** L'OMS a proposé des valeurs guides pour la concentration de l'arsenic dans l'eau de boisson. Cette valeur guide était de 200 µg/L, en 1958, et elle a progressivement été abaissée à 50 µg/L en 1993 puis à 10 µg/L actuellement. La plupart des pays industrialisés, notamment les Etats-Unis, l'Europe et le Japon, ont transposé cette valeur comme norme nationale. Le Canada a opté pour une valeur transitoire intermédiaire de 25 µg/L. De nombreux pays ont conservé la norme de 50 µg/L en partie en raison de la difficulté technique à doser en routine de faibles concentrations d'arsenic. Dans certains pays en voie de développement, l'arsenic ne figure pas sur la liste des substances à rechercher dans l'eau de boisson.

Sur la base de critères sanitaires, la valeur guide pour l'arsenic dans l'eau de boisson devrait être inférieure à 10 µg/L. Cette valeur guide est conditionnée par les limites de mesure. Il est donc nécessaire que les organismes de contrôle aient la possibilité technique de doser de faibles concentrations d'arsenic en routine.

De nombreux pays rencontrent des problèmes plus ou moins importants de contamination de leurs aquifères par de l'arsenic. Les pays les plus concernés sont l'Argentine, le Bangladesh, le Cambodge, le Chili, la Chine, les Etats-Unis d'Amérique, la Hongrie, le Mexique, la Roumanie, la Thaïlande et le Vietnam.

La déclaration de Taiyuran en 2004 sur la qualité de l'eau et l'arsenic mentionne qu'actuellement douze pays asiatiques sont affectés par des concentrations en arsenic dans les nappes phréatiques dépassant les limites admissibles. Au moins 50 millions de personnes sont exposées à des teneurs en arsenic de plus de 50 µg/L.

En Amérique latine, notamment en Argentine, en Bolivie, au Salvador, au Mexique, au Nicaragua et au Pérou, 4 millions de personnes vivant en zones rurales et consommant de l'eau de puits sont exposées à de l'eau de boisson contenant une concentration importante en arsenic.

L'Unicef, en collaboration avec le gouvernement du Bangladesh, a lancé un vaste programme de creusement de puits tubulaires pour fournir à la population une eau saine. Cette opération a conduit au forage de 8 à 12 millions de puits approvisionnant prés de 130 millions de personnes à travers le pays. Cette initiative a contribué à faire fortement reculer le taux de mortalité chez les nourrissons et les enfants de moins de cinq ans. Malheureusement, le sous-sol de ce pays est riche en arsenic et des analyses d'eaux ont révélé en 1993 que de nombreux puits étaient alimentés par une eau contaminée en arsenic. En fonction de leur localisation, tous les puits ne présentent pas le même niveau de contamination. De plus, la concentration en arsenic est susceptible d'évoluer dans le temps.

Ces problèmes ont souligné le manque de méthodes de dosage de l'arsenic simple à mettre en oeuvre et utilisables à grande échelle.

De nombreuses techniques de dosage de l'arsenic ont été développées. Ces techniques nécessitent l'utilisation d'appareils de laboratoire sophistiqués tels que des spectromètres d'absorption atomique ou des spectromètres de masse, ou bien correspondent à des kits de terrains (**[2]** et Hung D., Nekrassova O., Compton R. 2004. Analytical methods for inorganic arsenic in water: a review. Talanta 64:269-277 **[3]**).

Les normes en vigueur pour le dosage de l'arsenic dans l'eau de boisson préconisent l'utilisation de différentes méthodes spectroscopiques en fonction de la teneur attendue et des limites de détection souhaitée, à savoir : la spectrométrie d'absorption atomique par la technique des hydrures (AAS- technique des hydrures), la spectrométrie d'absorption atomique avec atomisation électrothermique (GF- AAS), la spectrométrie d'émission atomique couplée à une torche à plasma (ICP- Optique) et la spectrométrie de masse couplée à une torche à plasma (ICP- MS)

Ces méthodes possèdent des limites de détection inférieure à la valeur seuil de 10 µg/L mais nécessitent l'utilisation d'appareillages onéreux, fragiles, utilisables seulement en laboratoire par un personnel dûment formé. En outre, aucune de ces méthodes ne se prête à une analyse en routine d'un grand nombre d'échantillons.

En particulier, WO2010/004736 décrit une méthode et un kit de détermination de la présence d'arsenic dans un échantillon qui utilise un support solide sur lequel est immobilisé un fragment d'ADN comportant l'opéron ars. La protéine ArsR capable de se lier à l'opéron en absence d'arsenic est une protéine de fusion à la GFP qui est donc détectée directement par fluorescence.

Des kits de terrain de dosage de l'arsenic ont été développés et sont commercialisés. La technique de détection des kits de dosage de l'arsenic disponibles est basée sur l'apparition d'une coloration qui est, en général, appréciée à l'oeil nu. Ces tests peuvent être utilisés sur le terrain par du personnel non spécialisé mais présentent une limite de détection trop élevée pour l'analyse de l'eau ; à savoir 2 mg/L.

Il n'existe donc pas à l'heure actuelle de procédé d'analyse d'arsenic dans un échantillon étant à la fois simple à mettre en oeuvre, utilisable sur le terrain et ne nécessitant pas l'utilisation d'appareils sophistiqués, présentant une bonne sensibilité, permettant d'analyser un grand nombre d'échantillon et étant standardisable.

En outre, l'OMS précise sur son site Internet que « Mesurer la concentration d'arsenic dans l'eau de boisson à des niveaux importants pour la santé exige des analyses de laboratoire à l'aide de techniques et d'installations complexes et coûteuses ainsi que du personnel qualifié qu'il est difficile de trouver ou de financer dans bien des régions du monde. Les analyses de terrain permettent de déceler des concentrations élevées d'arsenic mais elles ne sont généralement pas fiables pour des concentrations inférieures importantes pour la santé humaine ».

Dans un rapport récent (EB118/14 du 24 mai 2006), l'OMS précise également que « L'inexistence d'un test simple, facile à mettre en oeuvre sur le terrain et peu onéreux continue d'être un obstacle important à une meilleure compréhension de l'étendue et de la gravité de la contamination de l'eau de boisson par l'arsenic et au développement des analyses locales de la qualité de l'eau dans les communautés ».

Il existe donc un réel besoin d'un procédé d'analyse d'arsenic dans un échantillon palliant ces défauts, inconvénients et obstacles de l'art antérieur, en particulier d'un procédé simple à mettre en oeuvre, standardisable, permettant d'analyser un grand nombre d'échantillon, ne nécessitant pas l'utilisation d'appareils sophistiqués et couteux tout en présentant une bonne sensibilité.

### Description de l'invention

La présente invention répond précisément à ces besoins et pallie aux défauts de l'art antérieur en fournissant un procédé et un kit d'analyse d'arsenic dans un échantillon.

Ainsi, la présente invention a notamment pour objet, un procédé d'analyse d'arsenic dans un échantillon comprenant, les étapes suivantes :
**(a)** mise en contact d'un échantillon susceptible de contenir de l'arsenic sur un support solide fonctionnalisé avec un fragment d'ADN comportant un site de fixation d'une protéine ArsR, site sur lequel est fixée une protéine ArsR, la présence d'arsenic provoquant la séparation de la protéine ArsR du fragment d'ADN ;
**(b)** incubation de l'échantillon en contact avec ledit support ;
**(c)** élimination de la protéine ArsR éventuellement séparée du fragment d'ADN par un lavage du support après l'incubation réalisée à l'étape **(b)** ; et
**(d)** analyse par une technique ELISA de la présence ou de l'absence de protéine ArsR restant fixée sur le fragment d'ADN.

Le procédé d'analyse d'arsenic selon la présente invention repose sur l'utilisation de la protéine de régulation (ArsR) des gènes de résistance à l'arsenic d'*Escherichia coli,* tel qu'*E*. *coli* K12 souche MG 1655 (ATCC 47076). La protéine ArsR possède deux sites de fixation : un site de fixation sur l'opéron *ars* et un site de fixation de l'arsenic.

Les bactéries sont fréquemment confrontées à la présence de métaux dans leur milieu et ont développé des systèmes de résistance (Mukhopadhyay R., Rosen B., Phung L., Silver S. 2002. Microbial arsenic: from geocycles to genes and enzymes. FEMS Microbiology Reviews 26:311-325 **[4]**). L'arsenic peut pénétrer dans les cellules bactériennes par le système de transport du phosphate. La plupart des bactéries résistent à ce composé en l'expulsant à l'extérieur de la cellule grâce à des pompes d'efflux. La transcription des gènes qui codent pour la synthèse de ces pompes d'efflux est réprimée en absence d'arsenic par la fixation de ArsR sur l'opéron *ars.* Les gènes qui composent cet opéron permettent aux bactéries de résister à la présence d'arsenic dans son environnement. La fixation de la protéine ArsR sur la région opératrice inhibe l'expression des gènes *ars* de ces bactéries. La fixation de l'arsenic sur ArsR induit un changement de conformation de cette protéine. Cette modification empêche la protéine ArsR de se fixer sur l'opérateur ou provoque le décrochement des protéines ArsR déjà fixées. Les bactéries peuvent ainsi réguler l'expression de leurs gènes de résistance à l'arsenic en fonction de la présence ou de l'absence d'arsenic dans leur milieu de croissance : en absence d'arsenic, la protéine ArsR se lie à la région opératrice de l'opéron *ars* et inhibe la transcription ; en présence d'arsenic, la protéine ArsR fixe ce composé et ne peut plus s'associer à l'ADN.

Les inventeurs ont extrait un fragment d'ADN et une protéine à partir de ces bactéries pour mettre au point le procédé de l'invention. Ils ont constaté que ces fragments d'ADN et protéine conservent leur activité.

Les inventeurs sont les tous premiers à avoir découvert, de manière surprenante, qu'une analyse par une technique ELISA de la présence ou de l'absence de protéine ArsR restant fixé sur un fragment d'ADN comportant un site de fixation d'une protéine ArsR permet d'analyser de l'arsenic dans un échantillon avec une excellente sensibilité, et permet de doser l'arsenic à des doses beaucoup plus faibles que les méthodes de l'art antérieur.

Par « échantillon », il faut comprendre toute substance susceptible de contenir de l'arsenic. Il peut s'agir par exemple d'un liquide ou d'un solide.

Lorsque l'échantillon est un liquide, il peut par exemple s'agir d'eau, d'une boisson, d'une peinture, d'un vernis etc.. Par exemple, la boisson peut être du lait, un jus de fruits, un soda, de l'eau aromatisée, un concentré de fruit. Lorsque l'échantillon est un liquide, il peut être dilué dans une solution aqueuse, c'est-à-dire dans une solution comprenant principalement ou exclusivement de l'eau.

Lorsque l'échantillon est un solide, il peut par exemple s'agir d'un aliment, de terre, de bois, de cendres etc.. Par exemple, l'aliment peut être un fruit, un légume, un plat préparé, de la viande, du poisson, de la farine, etc.. Lorsque l'échantillon est un solide, celui-ci est de préférence solubilisé au préalable afin de pouvoir analyser l'arsenic éventuellement présent dans celui-ci.

La solubilisation d'un échantillon solide peut être réalisée par tout procédé connu de l'homme du métier afin d'extraire l'arsenic éventuellement présent dans l'échantillon et le solubiliser. La solubilisation d'un échantillon solide peut par exemple consister en une étape d'immersion totale ou partielle de l'échantillon dans de l'eau et/ou une solution saline et/ou une solution acide permettant d'extraire l'arsenic de l'échantillon.

La solubilisation d'un échantillon solide peut également, par exemple, comprendre une étape choisie dans le groupe comprenant un broyage, un coupage, une agitation et une combinaison de ces étapes, en présence d'eau et/ou d'une solution saline et/ou d'une solution acide permettant d'extraire l'arsenic de l'échantillon.

On entend par « solution acide », toute solution dont le pH est inférieur à 7. La solution acide peut être par exemple toute solution répondant à la norme ISO 11466 :1995. Par exemple la solution acide peut comprendre tout acide permettant d'obtenir un pH inférieur à 7. Par exemple, l'acide peut être choisi dans le groupe comprenant l'acide chlorhydrique, l'acide fluorhydrique, l'acide nitrique, et un mélange de plusieurs de ces acides. De préférence, la solution acide comprend un mélange d'acide chlorhydrique et d'acide nitrique.

On entend par « solution saline », toute solution comprenant un sel. Par exemple, le sel est choisi dans le groupe comprenant NaCl, K₂HPO₄, KH₂PO₄, Na₂HPO, NaH₂PO₄ et un mélange de plusieurs de ces sels.

Par l'expression « mise en contact d'un échantillon susceptible de contenir de l'arsenic sur un support solide » il faut comprendre le fait de disposer, déposer, placer, mettre, poser ou projeter un échantillon susceptible de contenir de l'arsenic, à la surface d'un support solide.

L'arsenic que l'on cherche à analyser peut être sous toute forme permettant sa fixation sur la protéine ArsR. Il peut s'agir par exemple de la forme trivalente arsénite (As[III]) ou de la forme pentavalente arséniate (As[V]). Il peut s'agir également de l'antimoine (Sb) qui est un élément équivalent à l'arsenic.

L'expression « support solide », signifie tout support se présentant sous forme solide ou sous forme de gel. Selon la présente invention, le support peut par exemple être en membrane de nylon, en verre, en silicium, par exemple en silicium poreux, en gel d'agarose, en gel de polyacrylamide, en polystyrène, en polyéthylène ou en polypropylène. Le support solide peut par exemple être une plaque, une microplaque ou un tube. Par exemple, le support solide peut être une plaque ou une microplaque comprenant une pluralité de puits. L'avantage d'utiliser une plaque multi-puits est de pouvoir réaliser plusieurs tests en parallèle et/ou prévoir un certains nombre de puits pour effectuer des tests contrôles et/ou témoins.

Par l'expression « support solide fonctionnalisé » il faut comprendre tout support solide sur lequel est fixée une molécule. La molécule peut être par exemple un peptide, une protéine ou un fragment d'ADN, par exemple un fragment d'ADN comportant un site de fixation d'une protéine ArsR.

Selon le procédé de la présente invention, le support solide peut être fonctionnalisé par tout moyen connu de l'homme du métier afin de lier fortement une molécule au support solide, par exemple selon le protocole décrit dans Sagiv J. (1980) Journal of the American Chemical Society, 102, 92 **[5]**. Par exemple, la molécule peut être fixée par une méthode choisie dans le groupe comprenant la silanisation, l'hydrosilylation et l'électrochimie. Il peut s'agir par exemple d'une fonctionnalisation par liaison streptavidine-biotine. Il peut également s'agir de supports en polystyrène ou en polypropylène ayant reçu des traitements de surface pour faciliter la fixation de molécules organiques, par exemple choisis parmi PolySorp (marque de commerce), MediSorp (marque de commerce), MaxiSorp (marque de commerce), MultiSorp (marque de commerce) et CovaLink (marque de commerce), fabriqués par Nunc.

Par l'expression « fragment d'ADN comportant un site de fixation d'une protéine ArsR » on entend toute séquence d'acide nucléique sur lequel la protéine ArsR est susceptible de se fixer.

Le fragment d'ADN comportant un site de fixation d'une protéine ArsR peut être, par exemple, un promoteur du gène de résistance à l'arsenic d'*E*. *coli,* ou une partie du promoteur du gène de résistance à l'arsenic d'*E*. *coli* permettant la fixation d'une protéine ArsR. Par exemple le promoteur ou la partie du promoteur du gène de résistance à l'arsenic d'*E*. *coli,* est le promoteur du gène de résistance à l'arsenic d'*E*. *coli* K12 souche MG 1655, ou une partie du promoteur du gène de résistance à l'arsenic d'*E*. *coli* K12 souche MG 1655. Par exemple, la séquence du promoteur du gène de résistance à l'arsenic d'*E*. *coli* peut être choisi dans le groupe comprenant la séquence SEQ ID NO. 1, SEQ ID NO. 17 et une séquence active ayant au moins 80%, par exemple au moins 85%, par exemple au moins 90%, par exemple au moins 95 % d'identité avec SEQ ID NO. 1 ou SEQ ID NO. 17.

Les inventeurs ont en outre mis au point des séquences fragment d'ADN comportant un site de fixation d'une protéine ArsR qui sont particulièrement efficaces pour fixer la protéine ArsR dans la mise en oeuvre du procédé et du kit selon l'invention. Il s'agit des séquences SEQ ID NO. 23 et SEQ ID NO. 25 et de leur séquences complémentaires respective SEQ ID NO. 24 et SEQ ID NO. 26.

Ainsi, dans la présente, le fragment d'ADN comportant un site de fixation d'une protéine ArsR peut également être, par exemple, une séquence choisie dans le groupe comprenant SEQ ID NO. 23, SEQ ID NO. 24, SEQ ID NO. 25, SEQ ID NO. 26 et une séquence active ayant au moins 80%, par exemple au moins 85%, par exemple au moins 90%, par exemple au moins 95 % d'identité avec SEQ ID NO. 23, SEQ ID NO. 24, SEQ ID NO. 25 ou SEQ ID NO. 26. Ces séquences peuvent par exemple être obtenues par synthèse chimique (Eurofins MWG Opéron, Edersberg, Allemagne).

Par « séquence active », on entend une séquence qui permet la fixation de la protéine ArsR. Par exemple, la partie du promoteur du gène de résistance à l'arsenic d'*E*. *coli* peut avoir une taille inférieure à 100 paires de base.

La protéine ArsR possède un site de fixation à un fragment d'ADN comportant un site de fixation à la protéine ArsR et un site de fixation à l'arsenic. En l'absence d'arsenic, et lorsqu'un fragment d'ADN comportant un site de fixation à la protéine ArsR est présent, la protéine ArsR vient se fixer sur le site de fixation de la protéine ArsR du fragment d'ADN. On entend par « fixation de la protéine ArsR au fragment d'ADN », des interactions non covalentes, telles que des liaisons hydrogène, qui s'établissent entre la protéine ArsR et le fragment d'ADN en l'absence d'arsenic. En présence d'arsenic, celui-ci vient se fixer sur le site de fixation à l'arsenic de la protéine ArsR par des liaisons arsenic-thiol entre la molécule d'arsenic et les atomes de soufre des cystéines de la protéine ArsR. La fixation de l'arsenic sur le site de fixation à l'arsenic de la protéine ArsR induit un changement de conformation de la protéine ArsR qui provoque la séparation de la protéine ArsR du fragment d'ADN.

Selon la présente invention, la protéine ArsR peut être une protéine ArsR naturelle ou modifiée.

On entend par « protéine ArsR naturelle », une protéine ArsR obtenue à partir du gène codant pour cette protéine sans que la séquence du gène ou la protéine ArsR ait été modifiée. Par exemple, la protéine ArsR peut être obtenue à partir d'*E*. *coli* K12 souche MG 1655 par des techniques de purification bien connues de l'homme du métier (JC Janson, L Rydèn (1998). Protein purification: Principles, High-Resolution Methods, and Applications. Second edition, Wiley-VCH ISBN-10: 0-471-18626-0 **[6]**). La protéine ArsR obtenue à partir d'*E*. *coli* K12 souche MG 1655 a pour séquence SEQ ID NO. 4.

On entend par « protéine ArsR modifiée », une protéine ArsR obtenue à partir du gène codant pour cette protéine et dont la séquence a été modifiée, ou par modification post-traductionnelle de la protéine ArsR. Par exemple, la protéine ArsR peut être modifiée par recombinaison génétique, par exemple selon le protocole décrit dans F. Cordier-Ochsenbein *et al.* (Cordier-Ochsenbein, F., R. Guerois, et al. (1998). "Exploring the folding pathways of annexin I, a multidomain protein. II. Hierarchy in domain folding propensities may govern the folding process." J Mol Biol 279(5): 1177-85 **[7]**).

La protéine ArsR, peut par exemple être modifiée de manière à ce que les 20 derniers acides aminés composant sa séquence soit retirés (Xu et Rosen (1997) Dimerization is essential for DNA binding and repression by the ArsR metalloregulatory protein of Escherichia coli. J. Biol. Chem., Vol. 272, No. 25, pp. 15734-15738 **[8]**). L'utilisation d'une telle protéine ArsR tronquée permet de faciliter l'expression de la protéine ArsR sous forme soluble.

De manière complémentaire ou alternative aux modifications précitées, la protéine ArsR, peut par exemple être modifiée de manière à ce qu'elle soit liée de façon covalente avec un peptide de reconnaissance (« tag ») de séquence connue, pour la mise en oeuvre du test ELISA.

Le peptide de reconnaissance peut par exemple être synthétisé chimiquement, par exemple par synthèse chimique sur support solide, par exemple selon un procédé décrit dans « Fmoc Solid Phase Peptide Synthesis: A Practical Approach (Practical Approach Series) W. C. Chan (Editor), Peter D. White (Editor)", Oxford Univ Press 2000 » **[9]**.

Le peptide de reconnaissance peut être lié à la protéine ArsR par tout moyen connu de l'homme du métier. Par exemple, un peptide de reconnaissance peut être lié à la protéine ArsR par synthèse d'une séquence d'acides aminés correspondante à la succession de la séquence du peptide de reconnaissance et de la séquence de la protéine ArsR.

De préférence, le peptide de reconnaissance est lié de manière covalente, par exemple par une liaison peptidique, à la protéine ArsR.

Selon la présente invention, la séquence du peptide de reconnaissance peut être choisie parmi SEQ ID NO. 10, SEQ ID NO. 11, SEQ ID NO. 12, SEQ ID NO. 13, SEQ ID NO. 14, SEQ ID NO. 15, SEQ ID NO. 16 et une séquence permettant cette reconnaissance ayant au moins 80%, par exemple 85%, par exemple 90%, par exemple 95% d'identité avec ces séquences.

Les inventeurs ont mis au point des protéines ArsR modifiées qui sont particulièrement efficaces pour la mise en oeuvre du procédé et du kit selon l'invention. Il s'agit des protéines ArsR modifiées de séquences SEQ ID NO. 9 et SEQ ID NO. 22.

Selon l'invention, la protéine ArsR, naturelle ou modifiée, peut être une protéine ArsR de séquence choisie dans le groupe comprenant SEQ ID NO. 4, SEQ ID NO. 9, SEQ ID NO. 22 et une séquence permettant cette reconnaissance ayant au moins 80%, par exemple 85%, par exemple 90%, par exemple 95% d'identité avec ces séquences.

Selon le procédé de la présente invention, l'étape **(a)** de mise en contact peut être réalisée dans toute condition permettant une interaction entre l'arsenic susceptible d'être contenu dans l'échantillon et le support solide. Elle peut par exemple être réalisée à tout pH permettant l'interaction entre l'arsenic et la protéine ArsR. Par exemple, quelque soit la nature de l'échantillon, c'est-à-dire que l'échantillon soit un échantillon liquide ou solide, solubilisé dans de l'eau et/ou un solvant ou non, cet échantillon peut être mélangé avec une solution tampon afin de stabiliser le pH de l'échantillon. L'étape **(a)** de mise en contact peut, par exemple, être réalisée avec une solution tampon dont le pH est compris entre 6 et 8. De préférence, l'étape **(a)** de mise en contact est réalisée avec une solution dont le pH est de 7,4. Par exemple, la solution tampon est choisie dans le groupe comprenant le tampon phosphate salin (PBS ou « Phosphate Buffer Saline) et le tampon Tris salin (TBS ou « Tris Buffer Saline »).

Selon l'invention, l'étape **(b)** d'incubation peut être réalisée pendant toute durée et toute température permettant l'interaction entre l'arsenic et la protéine ArsR.

On entend par « incubation » le fait de laisser en contact l'échantillon avec le support solide. L'étape **(b)** d'incubation peut être réalisée dans toute condition permettant une interaction entre l'arsenic susceptible d'être contenu dans l'échantillon et le support solide. Elle peut par exemple être réalisée à toute température et toute durée permettant l'interaction entre l'arsenic et la protéine ArsR.

Selon l'invention, l'étape **(b)** d'incubation peut être réalisée pendant une durée allant de 1 minute à 90 minutes, par exemple de 30 à 90 minutes, par exemple de 40 à 80 minutes, par exemple de 50 à 70 minutes, par exemple 60 minutes.

Selon l'invention, l'étape **(b)** d'incubation peut être réalisée à une température comprise entre 15 et 37° C, par exemple entre 20 et 30° C, par exemple à 25° C.

Selon l'invention, l'étape **(c)** d'élimination de la protéine ArsR éventuellement séparé du fragment d'ADN peut être réalisée par tout moyen connue de l'homme du métier, permettant d'éliminer les protéines ArsR séparées du fragment d'ADN, sans détacher les protéines ArsR restant fixées sur le fragment d'ADN. Il peut s'agir par exemple de l'utilisation d'une solution de lavage. La solution de lavage peut par exemple être choisie dans le groupe comprenant de l'eau, par exemple de l'eau distillée et une solution tampon. Par exemple la solution tampon est choisie dans le groupe comprenant un PBS, une TBS, un PBS contenant 0,05% de Tween (PBST), et un TBS contenant 0,05% de Tween (TBST). L'homme du métier est à même de déterminer la quantité de solution de lavage et le nombre de lavage nécessaires.

On entend par « procédé d'analyse d'arsenic », tout procédé permettant de déterminer la présence ou l'absence d'arsenic et/ou de quantifier la quantité d'arsenic présent dans l'échantillon.

Selon le procédé de la présente invention, l'étape **(d)** d'analyse par une technique ELISA peut comprendre une étape d'observation de la présence ou de l'absence de protéine ArsR restant fixée sur un fragment d'ADN comportant un site de fixation d'une protéine ArsR. Le procédé d'analyse d'arsenic selon la présente invention peut aussi comprendre une étape de mesure de la quantité de protéine ArsR restant fixée sur ce fragment d'ADN.

On entend par « observation », le fait de regarder, par exemple à l'oeil nu ou au microscope, par exemple l'absence de coloration ou de luminescence, ou la coloration ou la luminescence par exemple produite par une réaction d'un substrat avec une enzyme, le substrat et l'enzyme étant définis ci-dessous. L'absence de coloration traduit la présence d'arsenic tandis qu'une coloration traduit l'absence d'arsenic dans l'échantillon analysé. L'observation peut par exemple être comparée avec une solution témoin comprenant de l'arsenic à une concentration déterminée ou avec une pluralité de solutions témoins comprenant de l'arsenic à différentes concentration déterminées.

L'observation peut en outre comprendre une étape de mesure de la coloration ou de la luminescence. Par exemple la coloration peut être mesurée à l'aide d'un spectrophotomètre. Par exemple, la luminescence peut être mesurée à l'aide d'un luminomètre.

Selon la présente invention, on entend par « technique ELISA (Enzyme Linked ImmunoSorbent Assay) », toute technique immuno-enzymatique de détection permettant de visualiser une réaction antigène-anticorps grâce à une réaction colorée produite par l'action sur un substrat d'une enzyme préalablement fixée à l'anticorps. De préférence, la technique ELISA est une technique ELISA directe ou indirecte.

Par « technique ELISA directe », on entend une technique faisant intervenir des anticorps conjugués avec une enzyme et dirigés contre la protéine ArsR ou vers un peptide de reconnaissance liée à la protéine ArsR.

Par « technique ELISA indirecte », on entend une technique faisant intervenir deux catégories d'anticorps. Les premiers anticorps sont dirigés contre la protéine ArsR ou vers un peptide de reconnaissance liée à la protéine ArsR, les seconds anticorps sont couplés avec une enzyme et dirigés contre les premiers anticorps.

Le principe des techniques ELISA peut par exemple être consulté dans ELISA, Methods in Molecular Biology, 1995, Volume 42, Part 1, 35-61, DOI: 10.1385/0-89603-279-5:35 **[10]**.

L'homme du métier est à même de déterminer quels anticorps, enzymes et substrat peuvent être utilisés.

Quelque soit la technique ELISA utilisée, l'anticorps dirigé contre la protéine ArsR peut être obtenu par un procédé décrit dans « Protocols for preparing immunogens, immunization of animais, and collection of antiserum may be found in Antibodies: A Laboratory Manual, E. Harlow and D. Lane, ed., Cold Spring Harbor Laboratory (Cold Spring Harbor, NY, 1988) pp. 55-120 » **[11]**.

Des exemples d'anticorps conjugués avec une enzyme et dirigés contre un peptide de reconnaissance lié à la protéine ArsR sont présentés dans le tableau 1 ci-dessous, associés aux séquences des peptides de reconnaissance correspondants.

**Tableau 1 : Peptide de reconnaissance et anticorps conjugués avec une enzyme associée**

| **Séquence du peptide de reconnaissance** | **SEQ ID NO.** | **Anticorps dirigé contre la séquence** | **Enzyme conjuguée** | **Référence produit et Fournisseurs** |
|---|---|---|---|---|
| DYKDDDDKG (Flag) | 10 | Anticorps anti-flag | Phosphatase alcaline | A9469 Sigma Aldrich |
| GKPIPNPLLGLDST (peptide v5) | 11 | Anticorps anti-V5 | Peroxydase de raifort (HRP) | V2260 Sigma Aldrich |
| HHHHHH (His) | 12 | Anticorps anti-polyhistidine | Phosphatase alcaline | A5588 Sigma Aldrich |
| EQKLISEEDL (c-MYC) | 13 | Anticorps anti-c-Myc | Phosphatase alcaline | A5963 Sigma Aldrich |
| YPYDVPDYA (HA) | 14 | Anticorps anti-HA | Phosphatase alcaline | A5477 Sigma Aldrich |
| YTDIEMNRLGK (VSV-G) | 15 | Anticorps anti-VSV-G | Peroxydase de raifort (HRP) | A5977 Sigma Aldrich |
| TDFYLK (AU5) | 16 | Anticorps anti-AU5 | Peroxydase de raifort (HRP) | NB600-462 Novus Biologicals Inc. |

Des exemples d'enzymes liées aux anticorps et de substrats correspondants pouvant être utilisés sont présentés dans le tableau 2 ci-dessous. Il existe différents types de substrats, à savoir des substrats chromogènes, chimioluminescents, et fluorescents.

**Tableau 2 : Enzymes et substrats associés**

| **Enzyme** | **Substrat** | **Référence produit et Fournisseurs** |
|---|---|---|
| Phosphatase alcaline | Alkaline Phosphatase Blue Microwell Substrate | AB0100 Sigma Aldrich |
| Phosphatase alcaline | Alkaline Phosphatase Red Microwell Substrate | AB0200 Sigma Aldrich |
| Phosphatase alcaline | 4-Nitrophenyl phosphate disodium salt hexahydrate (pNPP) | P4744-1 G Sigma Aldrich |
| Phosphatase alcaline | Disodium 2-chloro-5-(4-methoxyspiro {1,2-dioxetane-3,2'-(5'-chloro)tricyclo[3.3.1.13,7]decan}-4-yl) phenyl phosphate (CDP-Star) | 12 041 677 001 Roche Diagnostics |
| Peroxydase de raifort (HRP) | 3, 3', 5, 5' -tetramethylbenzidine (TMB) | T4444 Sigma Aldrich |
| Peroxydase de raifort (HRP) | Diammonium 2,2'-azino-bis(3-ethylbenzothiazoline-6-sulfonate | A9941 Sigma Aldrich |
| Peroxydase de raifort (HRP) | 5-Amino-2-hydroxybenzoic acid | A6178 Sigma Aldrich |
| Peroxydase de raifort (HRP) | Chemiluminescent Peroxidase Substrate | CPS260 Sigma Aldrich |
| Peroxydase de raifort (HRP) | 4-Methylumbelliferyl-phosphoric acid | M8883 Sigma Aldrich |

Selon l'invention, la meilleure sensibilité est obtenue avec les substrats fluorescents, puis les substrats chimioluminescents et enfin les substrats chromogènes. De préférence, les substrats utilisés en laboratoire d'analyse sont les substrats chimioluminescents ou fluorescents, car présentant une meilleure sensibilité. De préférence, les substrats utilisés sur le terrain sont des substrats chromogènes, car présentant une meilleure stabilité.

Selon le procédé de la présente invention, l'étape **(d)** d'analyse par une technique ELISA peut, par exemple, comprendre les étapes suivantes :
(i) ajout d'un anticorps dirigé contre la protéine ArsR, ledit anticorps étant conjugué à une enzyme, par exemple l'anticorps et l'enzyme étant ceux définis ci-dessus,
(ii) incubation de l'anticorps en contact avec la protéine ArsR,
(iii) élimination des anticorps qui ne se sont pas fixés sur la protéine ArsR par un lavage du support après l'incubation réalisée à l'étape (ii),
(iv) ajout d'un substrat produisant une coloration après avoir réagit avec l'enzyme, par exemple un substrat défini ci-dessus.

Selon l'invention, l'étape (ii) d'incubation peut être réalisée pendant toute durée et toute température permettant l'interaction entre la protéine ArsR et l'anticorps dirigé contre la protéine ArsR. Elle peut être réalisée par exemple pendant une durée allant de 1 minute à 90 minutes, par exemple de 30 à 90 minutes, par exemple de 40 à 80 minutes, par exemple de 50 à 70 minutes, par exemple 60 minutes. Elle peut être réalisée par exemple à une température comprise entre 15 et 37° C, par exemple entre 20 et 30° C, par exemple à 25° C.

Selon l'invention, l'étape (i) de mise en contact peut être réalisée à tout pH permettant l'interaction entre la protéine ArsR et l'anticorps dirigé contre la protéine ArsR. Par exemple, l'étape (i) de mise en contact peut être réalisée avec une solution tampon dont le pH est compris entre 6 et 8. De préférence, l'étape (i) de mise en contact est réalisée avec une solution dont le pH est de 7,4. La solution tampon peut être par exemple le tampon phosphate salin contenant 0,05% de Tween et contenant 1% (p/v) de solution de protéines (PBSTBr). Les protéines peuvent par exemple être choisies dans le groupe comprenant un « Blocking reagent » N° 11 096 176 001 (protéines de lait écrémé) (Roche Diagnostics GmbH), un « Blocking reagent » N° 11 112 589 001 (digestion protéolytique de gélatine purifiée) (Roche Diagnostics GmbH) et de l'albumine de sérum bovin (BSA).

Selon l'invention, l'étape (iii) d'élimination des anticorps qui ne se sont pas fixés sur la protéine ArsR peut être réalisée par toute solution de lavage connue de l'homme du métier, permettant d'éliminer les protéines ArsR séparées du fragment d'ADN, sans détacher les protéines ArsR restant fixées sur le fragment d'ADN. Il peut s'agir par exemple d'une solution choisie dans le groupe comprenant un PBS, une TBS, un PBS contenant 0,05% de Tween (PBST), et un TBS contenant 0,05% de Tween (TBST). L'étape (iii) d'élimination des anticorps qui ne se sont pas fixés sur la protéine ArsR peut être réalisée par exemple par application d'une solution de lavage sur le support solide. Un contrôle de lavage peut être réalisé en recherchant dans la solution tampon issu du lavage la présence de la protéine ArsR, par exemple au moyen d'anticorps dirigés contre la protéine ArsR ou un peptide de reconnaissance, lesdits anticorps étant définis ci-dessus.

Selon la présente invention, le procédé peut comprendre en outre une étape (e) de comparaison de la mesure réalisée à l'étape (d) avec un témoin comprenant de l'arsenic. Cette étape permet de comparer un échantillon avec une solution comprenant de l'arsenic à une concentration connue, permettant ainsi de déterminer si la concentration d'arsenic dans l'échantillon est supérieure, inférieure ou égale avec le témoin comprenant de l'arsenic. Par exemple le témoin comprenant de l'arsenic peut par exemple consister en une pluralité de solutions comprenant de l'arsenic à différentes concentrations, permettant de réaliser une gamme étalon.

Le procédé selon la présente invention peut en outre comprendre une étape **(f)** de comparaison de la mesure réalisée à l'étape **(d)** avec un témoin ne comprenant pas d'arsenic. Cette étape permet de vérifier que l'analyse ne présente pas de contaminations.

Selon un mode de réalisation particulier de l'invention, la protéine ArsR peut être fixée postérieurement à la fonctionnalisation du support solide avec un fragment d'ADN comportant un site de fixation d'une protéine ArsR. Par exemple, la protéine ArsR peut être ajoutée avant la mise en contact de l'échantillon susceptible de contenir de l'arsenic avec le support solide. La protéine ArsR peut également être ajoutée dans l'échantillon avant la mise en contact de l'échantillon sur le support solide.

La présente invention se rapporte également à un kit destiné à la détection d'arsenic dans un échantillon, ledit kit comprenant :
- un support solide fonctionnalisable ou fonctionnalisé avec un fragment d'ADN comprenant un site de fixation d'une protéine ArsR,
- un fragment d'ADN comprenant un site de fixation d'une protéine ArsR ;
- une protéine ArsR fixée sur ledit fragment d'ADN ;
- un moyen de détection par ELISA de la présence de la protéine ArsR.

Des exemples de support solide, de fragment d'ADN, et de protéine ArsR sont définis ci-dessus. Par exemple, le support solide peut être une plaque ou une microplaque comprenant une pluralité de puits.

Selon l'invention, le moyen de détection par ELISA de la présence de la protéine ArsR peut, par exemple, être un moyen de détection par colorimétrie ou luminescence. Il peut s'agir par exemple, des anticorps, enzymes et substrats définis ci-dessus.

Selon l'invention, le kit peut comprendre en outre de l'arsenic à une concentration déterminée. Par exemple, le kit comprend une pluralité de solutions comprenant de l'arsenic à différentes concentrations, permettant de réaliser une gamme étalon.

La présente invention fournit donc un procédé d'analyse d'arsenic dans un échantillon simple à mettre en oeuvre, standardisable, permettant d'analyser un grand nombre d'échantillon, ne nécessitant pas l'utilisation d'appareils sophistiqués et couteux tout en présentant une bonne sensibilité. La présente invention fournit également un kit de dosage de l'arsenic dans un échantillon permettant de mettre en oeuvre le procédé selon l'invention sur le terrain.

D'autres avantages pourront encore apparaître à l'homme du métier à la lecture des exemples ci-dessous, illustrés par les figures annexées, donnés à titre illustratif et non limitatif.

### Brève description des figures

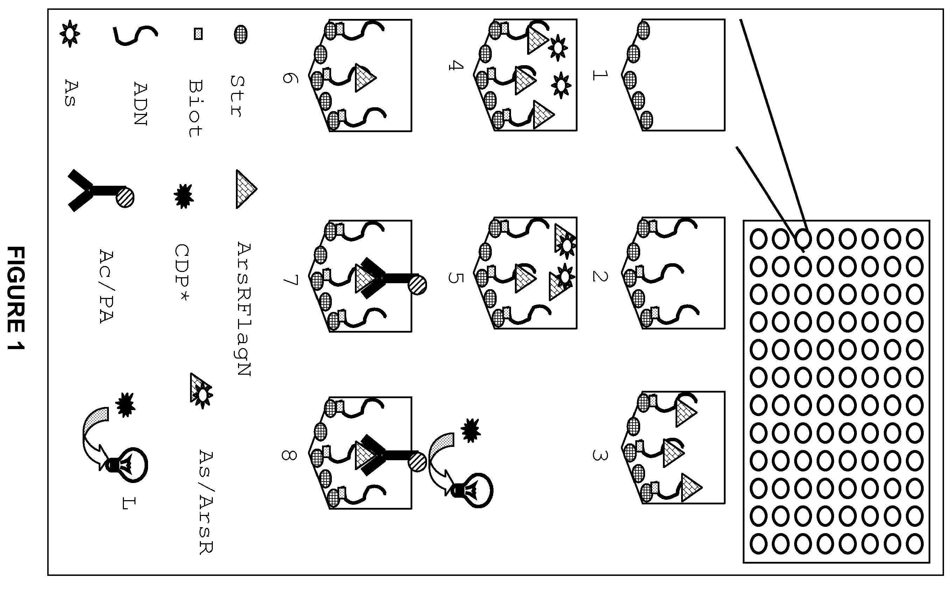

- La figure 1 représente le principe de la méthode immuno-enzymatique de dosage de l'arsenic conforme à la présente invention. En haut à droite est représentée une microplaque de 96 puits. En dessous sont représentés de manière agrandie, les puits de la microplaque en coupe transversale où : le puits (1) est fonctionnalisé avec de la streptavidine (« Str »), le puits où (2) un fragment d'ADN biotinylé comportant un site de fixation à la protéine ArsR est ajouté, le puits où (3) des protéines ArsR sur lesquelles sont fixées un peptide Flag en position N-terminal (« ArsRFIagN ») sont ajoutées et viennent se fixer sur les sites de fixation de la protéine ArsR du fragment d'ADN, le puits où (4) de l'arsenic (« As ») est ajouté, le puits où (5) l'arsenic se fixe sur des protéines ArsRFIagN qui se séparent du fragment d'ADN, le puits où (6) les protéines ArsRFIagN séparées et l'Arsenic sont éliminés, le puits où (7) des anticorps anti Flag couplés à la phosphatase alcaline (« Ac/PA ») sont ajoutés et viennent se fixer que sur les protéines ArsRFIagN restantes, et le puits où (8) un substrat de la phosphatase alcaline (« CEP* ») est ajouté, provoquant une émission de lumière.
   « Biot » signifie biotine, « As/ArsR » signifie arsenic fixé sur la protéine ArsR et « L » signifie émission de lumière lors de la transformation du substrat par la phosphatase alcaline.

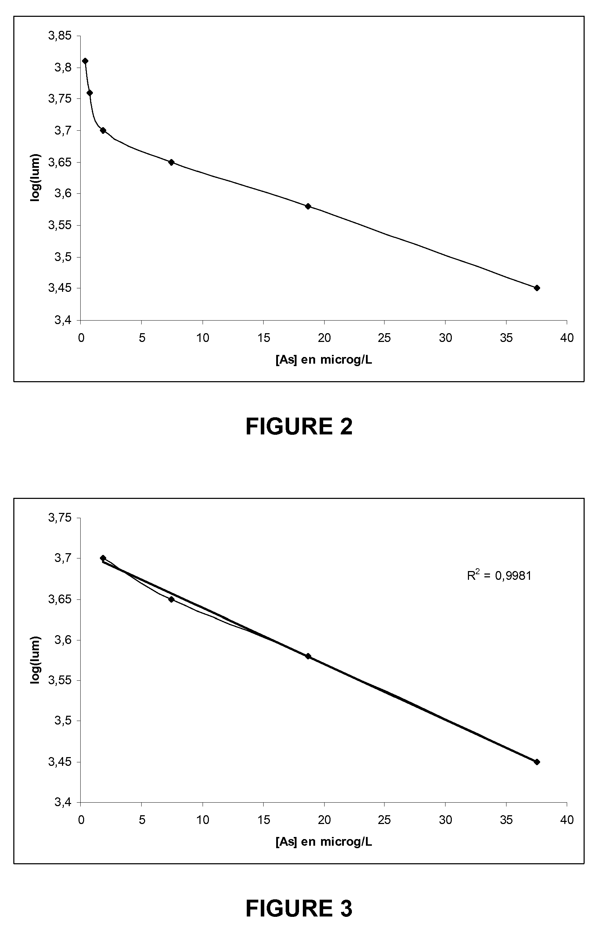

- La figure 2 représente une courbe modèle de la variation de luminescence en fonction de la concentration en arsenic (0,375 µg/L, 0,750 µg/L, 1,875 µg/L, 7,500 µg/L, 18,750 µg/L et 37,500 µg/L). L'abscisse représente la concentration en arsenic (« [As] ») exprimée en microgramme par litre (microg/L ou µg/L). L'ordonnée représente le logarithme de la valeur de luminescence (« log(lum) »).
- La figure 3 représente une courbe étalon de la variation de luminescence en fonction de la concentration en arsenic réalisée à partir de dilution d'arsenic à des concentrations connues (1,875 µg/L, 7,500 µg/L, 18,750 µg/L et 37,500 µg/L). L'abscisse représente la concentration en arsenic (« [As] ») exprimée en microgramme par litre (microg/L ou µg/L). L'ordonnée représente le logarithme de la valeur de luminescence (« log(lum) »). « R² » représente le coefficient de détermination multiple.

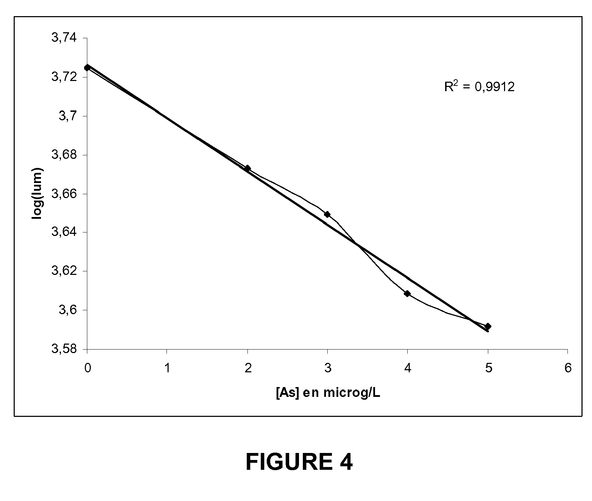

- La figure 4 représente une courbe étalon de la variation de luminescence en fonction de la concentration en arsenic réalisée à partir de dilution d'arsenic à des concentrations connues (0 µg/L, 2 µg/L, 3 µg/L, 4 µg/L, 5 µg/L). L'abscisse représente la concentration en arsenic (« [As] ») exprimée en microgramme par litre (microg/L ou µg/L). L'ordonnée représente le logarithme de la valeur de luminescence (« log(lum) »). « R² » représente le coefficient de détermination multiple.

### EXEMPLES

Dans les exemples ci-dessous, les compositions suivantes ont été utilisées :
- PBS : Phosphate, 10 mM ; NaCl, 137 mM ; KCI, 2 mM ; pH 7,4±0,1.
- PBST : PBS contenant 0,05% (v/v) de Tween 20.
- PBSTBr : PBST contenant 1% (p/v) de solution de « Blocking reagent » N° 11 096 176 001 (Roche Diagnostics GmbH) utilisée pour limiter la fixation non spécifique des anticorps sur le support.
- PBSTLE : PBST contenant 5% (p/v) de lait écrémé utilisé pour limiter la fixation non spécifique des protéines et des anticorps sur le support.
- CDP* : Substrat chimioluminescent de la phosphatase alcaline.
- Tampon 1 : TrisHCl, 100 mM ; NaCl, 100 mM ; MgCl2, 50 mM ; pH 9,5±0,1.

### Exemple 1 : Principe d'un procédé d'analyse conforme à la présente invention

Ce principe est illustré par la figure 1. Les chiffres entre parenthèses correspondent aux chiffres présentés dans cette figure 1.

Le détail de chacune de ces étapes est présenté dans les exemples 2, 3 et 4 ci-dessous.

La streptavidine est fixée au fond des puits de la microplaque (1).

Le fragment d'ADN est ajouté dans les puits de la microplaque (2). Les ADN non fixés sont éliminés par lavage.

L'extrait protéique brut contenant la protéine ArsR est ajouté dans les puits (3) pour permettre la fixation de la protéine sur son site sur l'ADN. Les protéines non fixées sont éliminées par lavage.

Différentes dilutions de la solution d'arsenic à doser sont ajoutées (4), l'arsenic se fixe sur la protéine ArsR (5) qui se détache de son site. Ces protéines sont éliminées par lavage. Seules les protéines ArsR qui n'ont pas fixé d'arsenic restent attachées à l'ADN (6).

Un anticorps spécifique de la séquence Flag est ajouté (7). Cet anticorps va se fixer sur la séquence Flag de la protéine ArsR liée à l'ADN. Cet anticorps est couplé à une enzyme, la phosphatase alcaline par exemple. Les anticorps non fixés sont éliminés par lavage.

Un substrat de l'enzyme est ajouté dans le puits. De la lumière est émise lorsque le substrat est clivé par l'enzyme (8). La quantité de lumière émise est proportionnelle à la quantité d'enzyme présente et donc à la quantité de protéine ArsR fixées sur l'ADN qui dépend de la teneur en arsenic. La quantité d'arsenic contenue dans l'échantillon est obtenue en comparant les résultats avec une courbe étalon.

### Exemple 2 : Fonctionnalisation de microplaque avec un fragment d'ADN comprenant un site de fixation de la protéine ArsR

Dans cet exemple, le fragment d'ADN contenant le site de fixation de la protéine ArsR utilisé est composé de 254 paires de bases (SEQ ID NO. 1, dénommé « Opars1 » dans la présente). La séquence Opars1 a été obtenue par amplification PCR (réaction de polymérisation en chaîne de l'ADN) à l'aide des amorces Opars1L (SEQ ID NO. 2) et Opars1 R (SEQ ID NO. 3). L'ADN chromosomique de la bactérie *Escherichia coli* K12 souche MG1655 (ATCC 47076) a servi de matrice pour l'amplification PCR. Une biotine a été ajoutée sur le nucléotide en position 5' lors de la synthèse de l'amorce Opars1 L. Des séquences Opars1 biotinylées, c'est-à-dire ayant une biotine en position 5', ont ainsi été obtenues.

Une microplaque « NUNC Immobilizer (marque de commerce) Streptavidin » (Microplaque Streptavidine Nunc, Dominique Dutscher (réf. 056212), Brumath, France) de 96 puits pouvant contenir un volume utile de 100 µl chacun et dont la surface est fonctionnalisée avec de la streptavidine, a été utilisée. Les puits de cette microplaque ont été lavés trois fois à l'aide de 300 µl de solution PBST.

Une solution comprenant des fragments d'ADN Opars1 a été diluée, dans de la solution PBS, pour obtenir une concentration d'ADN de 0,2 µM. 100 µl de cette solution ont été placés dans chaque puits de dosage de la microplaque « NUNC Immobilizer (marque de commerce) Streptavidin ». La microplaque a été incubée 60 minutes à 25°C, pour permettre la fixation de l'ADN dans les puits.

La biotine et la streptavidine possèdent une très forte affinité et sont capables de se lier spontanément. La présence d'une biotine sur la séquence Opars1 a donc permis la fixation de la séquence Opars1 à la surface des puits fonctionnalisés avec de la streptavidine.

L'ADN non fixé a été éliminé par trois lavages successifs avec 300 µl de PBST par puits.

### Exemple 3: Fixation d'une protéine ArsR sur la séquence Opars1 fonctionnalisée sur une microplaque

La protéine ArsR de séquence SEQ ID NO. 4 correspond à la protéine qui régule l'opéron Ars de *Escherichia coli* K12 souche MG1655. Le gène *arsR* de séquence SEQ ID NO. 5 codant cette protéine a été amplifié à partir de l'ADN chromosomique de cette souche à l'aide des amorces arsRR (SEQ ID NO. 6) et arsRL (SEQ ID NO. 7), et a été cloné dans le vecteur d'expression pQE70 (Qiagen) selon le protocole décrit dans le document « The QiaExpressionist : A handbook for high-level expression and purification of 6xHis-tagged proteins. June 2003. Fifth ed., Qiagen » **[12].** Une séquence Flag (SEQ ID NO. 10) a été insérée dans la région N-terminale de la protéine ArsR et a été nommée « ArsRFIagN » (SEQ ID NO. 9). La séquence Flag est reconnue par des anticorps anti-Flag (Ref. A9469, Sigma Aldrich, Saint-Quentin Fallavier, France). Cet ajout permettra de détecter et de doser plus efficacement la protéine ArsR.

Le plasmide pQE70 contenant le gène *arsRFlagN* (SEQ ID NO. 8) codant pour la protéine ArsRFIagN a été nommé « pQEarsRFlagN » et a été introduit dans la souche *Escherichia coli* M15[pREP4] (Qiagen SA, Courtaboeuf, France) afin de produire la protéine ArsRFlagN.

50 ml de milieu LB contenant 100 mg/L d'ampicilline ont été ensemencés avec une préculture de la souche M15[pREP4] / pQEarsRFlagN et ont été mis en agitation à 37°C.

Lorsque la densité optique de la culture a atteint une valeur de 0,5, la production de la protéine a été induite par ajout de 0,4 mM de IPTG (isopropyl β-D-1-thiogalactopyranoside) pendant 15 minutes. Cette durée de production de protéine a été choisie pour limiter la précipitation de la protéine ArsRFIagN dans des corps d'inclusion. Les cellules ont alors été récupérées par centrifugation à 4000 x *g* pendant 15 minutes à 4° C. Les cellules ont ensuite été lavées dans du tampon PBS. Le culot bactérien a été resuspendu dans 1 ml de tampon PBS. Puis, les cellules ont été lysées à l'aide d'un vibro-broyeur à bille (Vibro-broyeur MM301, Retsch) (« bead bitter ») qui est un système de lyse mécanique de cellules par agitation en présence de billes de verre de 0,1 mm de diamètre. L'extrait protéique a été centrifugé pendant 30 minutes à 30000 x *g* à 4° C pour éliminer les débris cellulaires. Le surnageant a été récupéré et stocké au froid dans de la glace pour être utilisé directement ou conservé au congélateur (-80° C).

La quantité de protéine ArsRFIagN soluble ainsi produite a été estimée à 200 µg.

Les 200 µg de protéines ArsRFIagN produits ont été dilués au 1/100 dans du tampon PBST. 100 µl de cette solution ont été ajoutés dans chacun des puits de dosage. La microplaque a été incubée 60 minutes à 25°C, pour permettre la fixation des protéines ArsRFIagN sur l'ADN dans les puits. La quantité de protéine ArsRFIagN ajoutée, par puits, a été estimée à environ 0,4 µg.

Les protéines non fixées ont été éliminées par trois lavages successifs avec 300 µl de PBST par puits.

### Exemple 4: Méthode immuno-enzymatique (ELISA) de dosage de l'arsenic

Un fragment d'ADN contenant le site de fixation de la protéine ArsR a été fixé sur un support de la même manière que présenté dans l'exemple 2 ci-dessus.

Une solution contenant la protéine ArsR a été ajoutée selon le protocole présenté dans l'exemple 3 ci-dessus, pour permettre la fixation de cette protéine sur l'ADN.

6 dilutions de solution d'arsenic, respectivement à 0,375 µg/L, 0,750 µg/L, 1,875 µg/L, 7,500 µg/L, 18,750 µg/L et 37,500 µg/L, ont été préparées dans du PBS afin d'établir une gamme étalon. 100 µl de chacune de ces dilutions ont été ajoutés respectivement dans un puits de la microplaque.

La microplaque a été incubée 60 minutes à 25° C, pour permettre la fixation de l'arsenic sur les protéines ArsRFlagN.

Les protéines ArsRFIagN non fixées ont été éliminées par trois lavages successifs avec 300 µl de PBST par puits.

100 µl d'une solution d'anticorps anti-Flag (Ref. A9469, Sigma Aldrich, Saint-Quentin Fallavier, France), conjugué à la phosphatase alcaline, ont été ajoutés dans chacun des puits. L'anticorps a préalablement été dilué dans du PBSTBr en fonction des recommandations du fournisseur. La microplaque est incubée 60 minutes à 25° C, pour permettre la fixation de l'anticorps sur les protéines ArsRFlagN.

Les anticorps anti-Flag non fixés ont été éliminés par trois lavages successifs avec 300 µl de PBST par puits.

Une solution de CEP* à 0,25 mM a été préparée dans du tampon 1. 100 µl de solution de CEP* ont été placés dans les différents puits de dosage. La lumière produite a immédiatement été mesurée à l'aide d'un luminomètre (LumiStar, BMG Lab Technologies).

Le principe de la méthode présentée dans les exemples 1 à 4 est illustré par la figure 1.

Les valeurs de luminescence (L) obtenues sont présentées dans le tableau 3 ci-dessous et illustré par les figures 2 et 3.

**Tableau 3 : Mesure de la luminescence en fonction de la concentration en arsenic**

| Concentration en AsIII (µM) | Concentration en AsIII (µg/L) | Valeur de Luminescence (L) | logL |
|---|---|---|---|
| 0,005 | 0,375 | 6426 | 3,81 |
| 0,01 | 0,750 | 5808 | 3,76 |
| 0,025 | 1,875 | 5023 | 3,70 |
| 0,100 | 7,500 | 4422 | 3,65 |
| 0,250 | 18,750 | 3776 | 3,58 |
| 0,500 | 37,500 | 2831 | 3,45 |

La courbe étalon obtenue est linéaire (figure 3) dès 1,875 µg/L d'arsenic, démontrant que la sensibilité de la technique est bien inférieure à la limite de détection de 10 µg/L des méthodes actuelles.

En outre le coefficient de détermination R² = 0,9981, qui est un indicateur permettant de juger la qualité d'une régression linéaire, notamment mesurant l'adéquation entre le modèle (figure 2) et les données observées (figure 3), démontre bien la fiabilité des résultats observés.

Jamais de tels résultats n'ont été obtenus. Le procédé selon la présente invention pourrait donc bien devenir le procédé de référence pour le dosage de l'arsenic dans un échantillon.

### Exemple 5 : Expression et purification d'une protéine ArsRFlagN-6His

La séquence du gène sauvage *arsR d'Escherichia coli* a été modifiée selon des méthodes décrites par Sorensen et al. ou Zhang et al. (Sorensen MA, Kurland CG, Pedersen S. (1989), Codon usage determines translation rate in Escherichia coli. J Mol Biol. 1989;207(2):365-77 **[13]** ; Zhang SP, Zubay G, Goldman E., Low-usage codons in Escherichia coli, yeast, fruit fly and primates. Gene. 1991;105(1):61-72 **[14]**) dans le but de permettre une meilleure expression de la protéine ArsR. Le gène de séquence SEQ ID NO. 18 a ainsi été synthétisé. Ce fragment d'ADN contient un site de restriction *Ncol* : CCATGG, la séquence Flag : GACTACAAAGACGACGACGACAAA (SEQ ID NO. 19), la séquence optimisée du gène *arsR* : GGTAGCTTTCTGCTGCCGATCCAGCTGTTCAAAATTCTGGCAGACGAA ACCCGTCTGGGTATTGTGTTACTGCTGAGCGAATTAGGCGAACTGTGC GTTTGCGATCTGTGTACCGCGTTAGATCAGAGTCAGCCGAAAATTAGC CGTCATCTGGCGTTATTACGCGAAAGCGGTCTGTTACTGGACCGTAAA CAGGGCAAATGGGTCCATTACCGCTTATCTCCGCATATTCCGGCTTGG GCAGCAAAAATCATTGACGAAGCCTGGCGTTGCGAACAGGAAAAAGTT CAGGCGATCGTCCGTAACCTGGCACGTCAAAATTGTAGCGGCGATAG CAAAAACATC (SEQ ID NO. 20) et un site *XhoI* : CTCGAG. Les sites de restriction *NcoI* et *XhoI* ont été introduits, respectivement, en début de séquence (région 5' du gène) et en fin de séquence (région 3'). Ces sites de restriction permettent de cloner le gène *arsR* dans le vecteur d'expression pET28b(+) commercialisée par la société Novagen (Cat. No. 70777, Merck, Lyons, France). Les vecteurs de la série pET ont été développés pour faciliter l'expression et la purification de protéines recombinantes dans *Escherichia coli* (Rosenberg, A.H., Lade, B.N., Chui, D., Lin, S., Dunn, J.J., and Studier, F.W. (1987) Vectors for selective expression of cloned DNAs by T7 RNA polymerase. Gene 56 (1): 125-135 **[15]**). Le gène de séquence SEQ ID NO. 18 a été cloné, dans le vecteur pET28b(+), sous la dépendance d'un promoteur fort du bactériophage T7 selon le protocole décrit dans la notice technique livrée avec le vecteur (pET System Manual, Novagen, TB055 8th Edition 02/99). La transcription du gène a été assurée par la polymérase T7 synthétisée par *E. coli* (souche hôte pour l'expression de la protéine recombinante). Cette polymérase n'a été synthétisée dans la souche *Escherichia coli* d'expression qu'en présence d'un inducteur : l'isopropyl β-D-1-thiogalactopyranoside (IPTG). Il a donc été possible de réguler finement le niveau d'expression de la protéine et d'obtenir un rendement élevé. Le vecteur pET28b(+) porte un gène de résistance à la kanamycine (sélection et maintien du plasmide) et deux séquences polyhistidine dans le site multiple de clonage. Ces séquences ont permis d'introduire une queue polyhistidine : HHHHHH (SEQ ID NO. 12) à partir de arsRFlagN après clonage dans le vecteur *pET-28b(*+*),* dans la région N- ou C-terminale de la protéine recombinante, ce qui facilitera la purification. Cette séquence polyhistidine peut se fixer sur une résine contenant du nickel ou du cobalt. La protéine recombinante a ainsi été retenue alors que les autres protéines sont éluées. La séquence du gène cloné dans le vecteur pET28b(+) et de la protéine arsRFlagN-6His correspondante sont présentées, respectivement, dans les séquences SEQ ID NO 21 et SEQ ID NO 22.

La construction a été introduite, par transformation, dans la bactérie *Escherichia coli* C43(DE3) pLysS (Miroux et Walker, Over-production of proteins in Escherichia coli: mutant hosts that allow synthesis of some membrane proteins and globular proteins at high levels. J Mol Biol. 1996;260(3):289-98 **[16])** (F - *ompT hsdSB (rB- mB-) gal dcm* (DE3) pLysS CmR) commercialisée par la société Lucigen (Euromedex, Souffelweyersheim, France).

Une culture de la nuit en milieu LB-Glucose (composition par litre de milieu : 10 g de tryptone, 5 g d'extraits autocatalytiques de levure, 5 g de NaCl, 10 g de glucose) additionné de 25 mg de kanamycine par litre de milieu a été utilisée pour inoculer un litre de milieu identique. Les bactéries ont été cultivées sous agitation, à 37°C, jusqu'à une densité optique de 0,5 à 600 nm. La production de protéine a alors été induite par l'addition de 0,5 mM d'IPTG. Les cellules induites ont été maintenues sous agitation, à 37°C, pendant 3 heures. Les cellules ont alors été récupérées par centrifugation (9 000 × *g*, 10 minutes, 4°C). Le culot est resuspendu dans du tampon PBS puis, les cellules ont été récupérées par centrifugation, comme précédemment. Les cellules ont été resuspendues dans du tampon PBS additionné de 10 mM d'imidazole (4 ml de tampon par gramme de culot bactérien). Un cocktail d'inhibiteurs de protéase (Protease Inhibitor cocktail Set III without EDTA, Cat. No. 539 134, Calbiochem, Merck, Lyons, France) a été ajouté (1 ml pour 40 ml de suspension bactérienne). Les cellules ont été lysées par une méthode mécanique (2 passages à 1500 bars dans un désintégrateur de cellules, modèle OneShot, Constant Systems Ltd., CeIID, Sauveterre, France). Le lysat a été centrifugé à 35 000 × g à 4°C pendant 30 minutes. Le surnageant a été récupéré puis clarifié par un passage sur filtre 0,22 µm.

La protéine ArsRFlagN-6His (SEQ ID NO. 22) a été purifiée par une chromatographie d'affinité (IMAC). Une colonne Ni-NTA a été équilibrée avec du tampon PBS + 10 mM d'imidazole. Le lysat clair a été déposé sur la colonne. Le tampon a été élué par gravité. La colonne a été lavée, une première fois, à l'aide de tampon PBS + 10 mM d'imidazole (10 volumes de colonne) puis, une seconde fois, à l'aide de tampon PBS + 20 mM d'imidazole. La protéine ArsR a été éluée à l'aide d'un gradient d'imidazole dans du tampon PBS (tampon PBS avec des concentrations croissantes d'imidazole : 30 à 400 mM). Les fractions collectées ont été analysées (gel SDS-PAGE). Les fractions qui contenaient la plus grande quantité de protéine ont été conservées à 4°C (3 ml de tampon PBS, imidazole 300 mM, pH 7,5). La concentration en protéine ArsR était de 1 mg/ml. La protéine est stable 15 jours à 4°C. Elle peut être conservée plusieurs semaines à des températures plus basses (-20 et -80°C) en présence d'un cryoprotecteur (glycérol à 50%, V/V).

### Exemple 6 : Fonctionnalisation de microplaque avec un fragment d'ADN comprenant un site de fixation de la protéine ArsR

Dans cet exemple, le fragment d'ADN contenant le site de fixation de la protéine ArsRFlagN-6His (SEQ ID NO. 22) utilisé est composé d'un fragment de 41 bases biotinylé en position 5' (SEQ ID NO. 25, dénommée « Pars2Lbio ») hybridé à un fragment d'ADN complémentaire de 33 bases (SEQ ID NO 26, dénommée « Pars2R »). Les séquences Pars2Lbio et Pars2R ont été obtenues par synthèse chimique (Eurofins MWG Opéron, Ebersberg, Allemagne).

L'oligonucléotide biotinylé (« Pars2Lbio ») a été utilisé à une concentration de 1 à 10 nM. L'oligonucléotide complémentaire (« Pars2R ») a été ajouté en excès pour favoriser la formation du double brin. Il a été dilués dans du tampon PBST (entre 1 à 10 nM, idéalement 5, pour l'oligonucléotide biotinylé ; entre 4 et 40 nM, idéalement 20, pour l'oligonucléotide complémentaire). Le mélange a été chauffé pendant une minute à 65°C pour éliminer les structures secondaires qu'auraient pu former chaque oligonucléotide.

Une microplaque « NUNC Immobilizer (marque de commerce) Streptavidin » (Microplaque Streptavidine Nunc, Dominique Dutscher (réf. 056212), Brumath, France) de 96 puits pouvant contenir un volume utile de 100 µl chacun et dont la surface est fonctionnalisée avec de la streptavidine, a été utilisée. Les puits de cette microplaque ont été lavés trois fois à l'aide de 300 µl de solution PBST.

La solution d'ADN a ensuite été distribuée dans les puits de la microplaque (100 µl par puits). La microplaque est placée pendant une heure à 25 °C pour permettre la fixation des fragments d'ADN grâce à l'affinité biotine - streptavidine.

La biotine et la streptavidine possèdent une très forte affinité et sont capables de se lier spontanément. La présence d'une biotine sur la séquence Opars1 a donc permis la fixation de la séquence Opars1 à la surface des puits fonctionnalisés avec de la streptavidine.

L'ADN non fixé a été éliminé par trois lavages successifs avec 300 µl de PBST par puits.

L'utilisation des SEQ ID NO. 23 (« Pars1Lbio ») et SEQ ID NO. 24 (« Pars1R »), à la place des séquences respectives SEQ ID NO. 25 (« Pars2Lbio ») et SEQ ID NO 26 (« Pars2R »), est également possible.

### Exemple 7 : Fixation d'une protéine ArsRFlagN-6His sur la séquence Pars2LRbio fonctionnalisée sur une microplaque

100 µl de solution PBSTLE ont été ajoutés dans les puits de la microplaque. La microplaque est incubée 60 minutes à 25°C pour permettre la fixation non spécifique des protéines de lait sur le fond et les parois du puits. Cette étape limite les fixations non spécifiques de la protéine ArsRFlagN-6His (SEQ ID NO. 22) et des anticorps conjugués anti-Flag (Ref. A9469, Sigma Aldrich, Saint-Quentin Fallavier, France). Les protéines non fixées ont été éliminées par trois lavages successifs des puits avec 300 (µl de tampon PBST.

Une solution de protéine ArsRFlagN-6His (SEQ ID NO. 22) à 1 µg/ml a été préparée dans du tampon PBST. 100 µl de cette solution ont été ajoutés dans chacun des puits de dosage. La microplaque a été incubée 60 minutes à 25°C, pour permettre la fixation des protéines ArsRFlagN-6His sur l'ADN dans les puits.

Les protéines non fixées ont été éliminées par trois lavages successifs avec 300 µl de PBST par puits.

### Exemple 8 : Méthode immuno-enzymatique (ELISA) de dosage de l'arsenic

Le fragment d'ADN défini dans l'exemple 6 et contenant le site de fixation de la protéine ArsR a été fixé sur un support de la même manière que présenté dans l'exemple 6 ci-dessus.

Une solution contenant la protéine ArsRFlagN-6His (SEQ ID NO. 22) a été ajoutée selon le protocole présenté dans l'exemple 7 ci-dessus, pour permettre la fixation de cette protéine sur l'ADN.

5 dilutions de solution d'arsenic, respectivement à 0 µg/L, 2 µg/L, 3 µg/L, 4 µg/L, et 5 µg/L, ont été préparées dans du PBS afin d'établir une gamme étalon. 100 µl de chacune de ces dilutions ont été ajoutés respectivement dans un puits de la microplaque.

La microplaque a été incubée 60 minutes à 25° C, pour permettre la fixation de l'arsenic sur les protéines ArsRFlagN-6His (SEQ ID NO. 22).

Les protéines ArsRFlagN-6His (SEQ ID NO. 22) non fixées ont été éliminées par trois lavages successifs avec 300 µl de PBST par puits.

100 µl d'une solution d'anticorps anti-Flag (Ref. A9469, Sigma Aldrich, Saint-Quentin Fallavier, France), conjugué à la phosphatase alcaline, ont été ajoutés dans chacun des puits. L'anticorps a préalablement été dilué dans du PBST en fonction des recommandations du fournisseur (facteur de dilution 1/500). La microplaque est incubée 60 minutes à 25° C, pour permettre la fixation de l'anticorps sur les protéines ArsRFlagN-6His.

Les anticorps anti-Flag non fixés ont été éliminés par trois lavages successifs avec 300 µl de PBST par puits.

Une solution de CEP* à 0,25 mM a été préparée dans du tampon 1. 100 µl de solution de CEP* ont été placés dans les différents puits de dosage. La lumière produite a immédiatement été mesurée à l'aide d'un luminomètre (LumiStar, BMG Lab Technologies).

Les valeurs de luminescence (L) obtenues sont présentées dans le tableau 4 ci-dessous et illustré par la figure 4.

**Tableau 4 : Mesure de la luminescence en fonction de la concentration en arsenic**

| Concentration en AsIII (µM) | Concentration en AsIII (µg/L) | Valeur de Luminescence (L) | logL |
|---|---|---|---|
| 0 | 0 | 5308 | 3,7249 |
| 0.027 | 2 | 4710 | 3,6730 |
| 0.040 | 3 | 4457 | 3,6490 |
| 0.053 | 4 | 4061 | 3,6086 |
| 0.067 | 5 | 3906 | 3,5917 |

La courbe étalon obtenue est linéaire (figure 4), démontrant que la sensibilité de la technique est bien inférieure à la limite de détection de 10 µg/L des méthodes actuelles.

En outre le coefficient de détermination *R*² = 0,9981, qui est un indicateur permettant de juger la qualité d'une régression linéaire démontre bien la fiabilité des résultats observés.

Jamais de tels résultats n'ont été obtenus, notamment à de si faibles concentrations.

### Listes des références

**[1]** Bisson M., Houeix N., Hulot C., Lacroix G., Lefevre J.P., Leveque S., Magaud H., Morin A. 2006. Arsenic et ses dérivés inorganiques. INERIS - DRC-01-25590-00DF258.doc
**[2]** Organisation Mondiale de la Santé. 2006. Réduction des concentrations d'arsenic et sécurité de la nappe phréatique. EB118/14.
**[3]** Hung D., Nekrassova O., Compton R. 2004. Analytical methods for inorganic arsenic in water: a review. Talanta 64:269-277.
**[4]** Mukhopadhyay R., Rosen B., Phung L., Silver S. 2002. Microbial arsenic: from geocycles to genes and enzymes. FEMS Microbiology Reviews 26:311-325.
**[5]** Sagiv J. (1980) Journal of the American Chemical Society, 102, 92
**[6]** JC Janson, L Rydèn (1998). Protein purification: Principles, High-Resolution Methods, and Applications. Second édition, Wiley-VCH ISBN-10: 0-471-18626-0.
**[7]** Cordier-Ochsenbein, F., R. Guerois, et al. (1998). "Exploring the folding pathways of annexin I, a multidomain protein. II. Hierarchy in domain folding propensities may govern the folding process." J Mol Biol 279(5): 1177-85.
**[8]** Xu et Rosen (1997) Dimerization is essential for DNA binding and repression by the ArsR metalloregulatory protein of Escherichia coli. J. Biol. Chem., Vol. 272, No. 25, pp. 15734-15738.
**[9]** Fmoc Solid Phase Peptide Synthesis: A Practical Approach (Practical Approach Séries) W. C. Chan (Editor), Peter D. White (Editor)", Oxford Univ Press 2000.
**[10]** ELISA, Methods in Molecular Biology, 1995, Volume 42, Part 1, 35-61, DOI : 10.1385/0-89603-279-5:35.
**[11]** Protocols for preparing immunogens, immunization of animais, and collection of antiserum may be found in Antibodies: A Laboratory Manual, E. Harlow and D. Lane, ed., Cold Spring Harbor Laboratory (Cold Spring Harbor, NY, 1988) pp. 55-120.
**[12]** The QiaExpressionist : A handbook for high-level expression and purification of 6xHis-tagged proteins. June 2003. Fifth ed., Qiagen.
[13] Sorensen MA, Kurland CG, Pedersen S. (1989), Codon usage determines translation rate in Escherichia coli. J Mol Biol. 1989;207(2):365-77.
[14] Zhang SP, Zubay G, Goldman E., Low-usage codons in Escherichia coli, yeast, fruit fly and primates. Gene. 1991;105(1):61-72.
[15] Rosenberg, A.H., Lade, B.N., Chui, D., Lin, S., Dunn, J.J., and Studier, F.W. (1987) Vectors for selective expression of cloned DNAs by T7 RNA polymerase. Gene 56 (1): 125-135
[16] Miroux et Walker, Over-production of proteins in Escherichia coli: mutant hosts that allow synthesis of some membrane proteins and globular proteins at high levels. J Mol Biol. 1996;260(3):289-98.

## Revendications

1. Procédé d'analyse d'arsenic dans un échantillon comprenant, les étapes suivantes :
**(a)** mise en contact d'un échantillon susceptible de contenir de l'arsenic sur un support solide fonctionnalisé avec un fragment d'ADN comportant un site de fixation d'une protéine ArsR, site sur lequel est fixée une protéine ArsR, la présence d'arsenic provoquant la séparation de la protéine ArsR du fragment d'ADN;
**(b)** incubation de l'échantillon en contact avec ledit support ;
**(c)** élimination de la protéine ArsR éventuellement séparée du fragment d'ADN par un lavage du support après l'incubation réalisée à l'étape **(b)** ; et
**(d)** analyse par une technique ELISA de la présence ou de l'absence de protéine ArsR restant fixée sur le fragment d'ADN.

2. Procédé selon la revendication 1, ledit procédé comprenant en outre une étape **(e)** de comparaison de la mesure réalisée à l'étape **(d)** avec un témoin comprenant de l'arsenic.

3. Procédé selon la revendication 1 ou 2, dans lequel la fonctionnalisation du fragment d'ADN sur le support solide est réalisée par liaison streptavidine-biotine.

4. Procédé selon l'une quelconque des revendications 1 à 3, dans lequel l'étape **(b)** d'incubation est réalisée pendant une durée allant de 1 minute à 90 minutes.

5. Procédé selon l'une quelconque des revendications 1 à 4, dans lequel l'étape **(b)** d'incubation est réalisée à une température comprise entre 15° C et 37 °C.

6. Procédé selon l'une quelconque des revendications 1 à 5, dans lequel l'étape **(a)** de mise en contact est réalisée avec une solution tampon dont le pH est compris entre 6 et 8.

7. Procédé selon l'une quelconque des revendications 1 à 6, dans lequel le fragment d'ADN comportant un site de fixation d'une protéine ArsR est un promoteur du gène de résistance à l'arsenic d'*E*. *coli,* ou une partie du promoteur du gène de résistance à l'arsenic d'*E*. *coli* permettant la fixation d'une protéine ArsR.

8. Procédé selon l'une quelconque des revendications 1 à 6, dans lequel la séquence du fragment d'ADN comportant un site de fixation d'une protéine ArsR est SEQ ID NO. 1, SEQ ID NO. 17, SEQ ID NO. 23, SEQ ID NO. 24, SEQ ID NO. 25, SEQ ID NO. 26 ou une séquence ayant au moins 80% d'identité avec SEQ ID NO. 1, SEQ ID NO. 17, SEQ ID NO. 23, SEQ ID NO. 24, SEQ ID NO. 25 ou SEQ ID NO. 26.

9. Procédé selon l'une quelconque des revendications 1 à 8, dans lequel le support solide est en en polystyrène, en polyéthylène ou en polypropylène.

10. Procédé selon l'une quelconque des revendications 1 à 9, dans lequel la protéine ArsR est liée de façon covalente avec un peptide de reconnaissance de séquence connu, pour la mise en oeuvre du test ELISA.

11. Procédé selon la revendication 10, dans lequel la séquence du peptide de reconnaissance est choisie parmi SEQ ID NO. 10, SEQ ID NO. 11, SEQ ID NO. 12, SEQ ID NO. 13, SEQ ID NO. 14, SEQ ID NO. 15, SEQ ID NO. 16 et une séquence ayant au moins 80% d'identité avec ces séquences.

12. Kit destiné à la détection d'arsenic dans un échantillon, ledit kit comprenant :
- un support solide fonctionnalisable ou fonctionnalisé avec un fragment d'ADN comprenant un site de fixation d'une protéine ArsR,
- un fragment d'ADN comprenant un site de fixation d'une protéine ArsR ;
- une protéine ArsR fixée sur ledit fragment d'ADN ;
- un moyen de détection par ELISA de la présence de la protéine ArsR.

13. Kit selon la revendication 12, dans lequel le moyen de détection par ELISA de la présence de la protéine ArsR, est un moyen de détection par colorimétrie ou luminescence.

14. Kit selon la revendication 12 ou 13, comprenant en outre de l'arsenic à une concentration déterminée.

15. Kit selon l'une quelconque des revendications 12 à 14, dans lequel le support solide est une plaque ou une microplaque comprenant une pluralité de puits.

## Patentansprüche

1. Verfahren zur Analyse von Arsen in einer Probe, das folgende Schritte umfasst:
(a) auf einem funktionalisierten festen Träger Inkontaktbringen einer Probe, die vermutlich Arsen enthält, mit einem DNA-Fragment, das eine Bindungsstelle für ein ArsR-Protein aufweist, an die ein ArsR-Protein gebunden ist, wobei das Vorhandensein von Arsen die Trennung des ArsR-Proteins von dem DNA-Fragments verursacht;
b) Inkubation der Probe im Kontakt mit dem Träger;
(c) Entfernen des gegebenenfalls von dem DNA-Fragment abgetrennten ArsR-Proteins durch Waschen des Trägers nach der in Schritt (b) durchgeführten Inkubation; und
(d) Analyse durch eine ELISA-Technik des Vorhandenseins bzw. Fehlens des ArsR-Proteins, das an das DNA-Fragment gebunden bleibt.

2. Verfahren nach Anspruch 1, wobei das Verfahren ferner einen Schritt (e) zum Vergleichen der in Schritt (d) durchgeführten Messung mit einem Arsen umfassenden Muster umfasst.

3. Verfahren nach Anspruch 1 oder 2, wobei die Funktionalisierung des DNA-Fragments auf dem festen Träger durch Biotin-Streptavidin-Bindung gebildet wird.

4. Verfahren nach einem der Ansprüche 1 bis 3, wobei der Schritt (b) der Inkubation während einer Dauer von 1 Minute bis 90 Minuten durchgeführt wird.

5. Verfahren nach einem der Ansprüche 1 bis 4, wobei der Schritt (b) der Inkubation bei einer Temperatur zwischen 15 °C und 37 °C durchgeführt wird.

6. Verfahren nach einem der Ansprüche 1 bis 5, wobei Schritt (a) des Inkontaktbringens mit einer Pufferlösung mit einem pH-Wert zwischen 6 und 8 durchgeführt wird.

7. Verfahren nach einem der Ansprüche 1 bis 6, wobei das DNA-Fragment, das eine Bindungsstelle eines ArsR-Protein aufweist, ein Promotor des Gens für die Resistenz gegen Arsen von *E. coli* ist, oder ein Teil des Promotors des Gens für die Resistenz gegen Arsen von *E. coli* ist, der es ermöglicht ein ArsR-Protein zu binden.

8. Verfahren nach einem der Ansprüche 1 bis 6, wobei die Sequenz des DNA-Fragments, die eine Bindungsstelle eines ArsR-Proteins umfasst, die SEQ ID NO. 1, SEQ ID NO. 17, SEQ ID NO. 23, SEQ ID NO. 24, SEQ ID NO. 25, SEQ ID NO. 26 ist, oder eine Sequenz, die mindestens zu 80% mit SEQ ID NO. 1, SEQ ID NO. 17, SEQ ID NO. 23, SEQ ID NO. 24, SEQ ID NO. 25 oder SEQ ID NO. 26 identisch ist.

9. Verfahren nach einem der Ansprüche 1 bis 8, wobei der feste Träger aus Polystyrol, Polyethylen oder Polypropylen ist.

10. Verfahren nach einem der Ansprüche 1 bis 9, wobei das ArsR-Protein für die Durchführung des ELISA-Test kovalent an ein Erkennungspetid mit bekannter Sequenz gebunden ist.

11. Verfahren nach Anspruch 10, wobei die Sequenz des Erkennungspeptids aus SEQ ID NO 10, SEQ ID NO. 11 SEQ ID NO. 12, SEQ ID NO. 13, SEQ ID NO. 14, SEQ ID NO. 15, SEQ ID NO. 16 ausgewählt ist und eine Sequenz mindestens zu 80% mit diesen Sequenzen identisch ist.

12. Ein Kit zum Nachweis von Arsen in einer Probe, wobei der Kit Folgendes umfasst:
- einen funktionalisierbaren oder funktionalisierten festen Träger mit einem DNA-Fragment, das eine Bindungsstelle eines ArsR-Proteins umfasst,
- ein DNA-Fragment, das eine Bindungsstelle eines ArsR-Proteins umfasst;
- ein an das DNA-Fragment gebundenes ArsR-Protein;
- ein Erfassungsmittel des Vorhandenseins des ArsR-Proteins durch ELISA.

13. Kit nach Anspruch 12, wobei das Erfassungsmittel des Vorhandenseins des ArsR-Proteins durch ELISA ein Erfassungsmittel durch Farbmetrik oder Lumineszenz ist.

14. Kit nach Anspruch 12 oder 13, ferner Arsen in einer bestimmten Konzentration umfassend.

15. Kit nach einem der Ansprüche 12 bis 14, wobei der feste Träger eine Platte oder eine Mikroplatte ist, die eine Vielzahl von Vertiefungen umfasst.

## Claims

1. A process for analyzing arsenic in a sample, comprising the following steps:
**(a)** contacting a sample suspected of containing arsenic on a solid support functionalized with a DNA fragment comprising an ArsR protein binding site, site on which an ArsR protein is attached, the presence arsenic causing separation of the ArsR protein from the DNA fragment;
**(b)** incubation of the sample in contact with the said support;
**(c)** elimination of ArsR protein separated from the DNA fragment by washing the support after the incubation carried out in step (b); and
**(d)** analyzing by an ELISA technique to detect the presence or absence of ArsR protein remaining attached to the DNA fragment.

2. The process according to claim 1, said process further comprising a step **(e)** of comparison of the measurement made in step **(d)** with a control sample containing arsenic.

3. The process according to claim 1 or 2, wherein the functionalization of the DNA fragment on the solid support is achieved by streptavidin-biotin bond.

4. The process according to anyone of claims 1 to 3, wherein the incubation step **(b)** is carried out for a time period ranging from 1 minute to 90 minutes.

5. The process according to anyone of claims 1 to 4, wherein the incubation step **(b)** is carried out at a temperature ranging from 15°C to 37°C.

6. The process according to anyone of claims 1 to 5, wherein the contacting step **(a)** is carried out with a buffer solution whose pH level is comprised between 6 and 8.

7. The process according to anyone of claims 1 to 6, wherein the DNA fragment containing an ArsR protein binding site is a promoter of the arsenic resistance gene of *E. coli*, or a part of the promoter of the arsenic resistance gene of *E. coli* enabling binding of an ArsR protein.

8. The process according to anyone of claims 1 to 6, wherein the sequence of the DNA fragment containing an ArsR protein binding site is SEQ ID NO. 1, SEQ ID NO. 17, SEQ ID NO. 23, SEQ ID NO. 24, SEQ ID NO. 25, SEQ ID NO. 26 or a sequence having at least 80% of identity with SEQ ID NO. 1, SEQ ID NO. 17, SEQ ID NO. 23, SEQ ID NO. 24, SEQ ID NO. 25 or SEQ ID NO. 26.

9. The process according to anyone of claims 1 to 8, wherein the solid support is made of polystyrene, polyethylene or polypropylene.

10. The process according to anyone of claims 1 to 9, wherein the ArsR protein is covalently bound to a known sequence of a recognition peptide, for the implementation of the ELISA test.

11. The process according to claim 10, wherein the sequence of the recognition peptide is selected from among SEQ ID NO. 10, SEQ ID NO. 11, SEQ ID NO. 12, SEQ ID NO. 13, SEQ ID NO. 14, SEQ ID NO. 15, SEQ ID NO. 16 and a sequence having at least 80% of identity with these sequences.

12. A kit intended for the detection of arsenic in a sample, said kit comprising:
- a solid support functionalizable or functionalized with a DNA fragment comprising an ArsR protein binding site;
- a DNA fragment containing an ArsR protein binding site;
- an ArsR protein fixed onto said DNA fragment;
- a means for detection by ELISA of the presence of the ArsR protein.

13. The kit according to claim 12, wherein the means for detection by ELISA of the presence of the ArsR protein, is a detection means using colorimetry or luminescence.

14. The kit according to claim 12 or 13, further comprising arsenic at a determined concentration.

15. The kit according to anyone of claims 12 to 14, wherein the solid support is a plate or a microplate containing a plurality of wells.


### SEQUENCE LISTING

<110> Université de Lorraine
<120> DOSAGE D'ARSENIC PAR ELISA,
<130> BNT208074PC00
<150> FR1151566
   <151> 2011-02-25
<160> 26
<170> PatentIn version 3.5
<210> 1
   <211> 254
   <212> DNA
   <213> Artificial Sequence
<220>
   <223> Opars1 : région opératrice de l'opéron ars d'Escherichia coli
<400> 1
   accaactcag ggctggaaag taaaaaaccg acgcaaagtc ggttttttta cgtcctgatt        60
   cagacctcct ttcaaatgaa tagccaactc aaaattcaca cctattacct tcctctgcac       120
   ttacacattc gttaagtcat atatgttttt gacttatccg cttcgaagag agacactacc       180
   tgcaacaatc aggagcgcaa tatgtcattt ctgttaccca tccaattgtt caaaattctt       240
   gctgatgaaa cccg                                                         254
<210> 2
   <211> 19
   <212> DNA
   <213> Artificial Sequence
<220>
   <223> Amorce Opars1L
<400> 2
   accaactcag ggctggaaa 19
<210> 3
   <211> 20
   <212> DNA
   <213> Artificial Sequence
<220>
   <223> Amorce Opars1R
<400> 3
   cgggtttcat cagcaagaat 20
<210> 4
   <211> 117
   <212> PRT
   <213> Artificial Sequence
<220>
   <223> Séquence de la protéine ArsR d'Escherichia coli K12 souche MG1655
<400> 4
   Met Ser Phe Leu Leu Pro Ile Gln Leu Phe Lys Ile Leu Ala Asp Glu
   1               5                   10                  15
   Thr Arg Leu Gly Ile Val Leu Leu Leu Ser Glu Leu Gly Glu Leu Cys
               20                  25                  30
   Val Cys Asp Leu Cys Thr Ala Leu Asp Gln Ser Gln Pro Lys Ile Ser
           35                  40                  45
   Arg His Leu Ala Leu Leu Arg Glu Ser Gly Leu Leu Leu Asp Arg Lys
       50                  55                  60
   Gln Gly Lys Trp Val His Tyr Arg Leu Ser Pro His Ile Pro Ala Trp
   65                  70                  75                  80
   Ala Ala Lys Ile Ile Asp Glu Ala Trp Arg Cys Glu Gln Glu Lys Val
                   85                  90                  95
   Gln Ala Ile Val Arg Asn Leu Ala Arg Gln Asn Cys Ser Gly Asp Ser
               100                 105                 110
   Lys Asn Ile Cys Ser
           115
<210> 5
   <211> 384
   <212> DNA
   <213> Artificial Sequence
<220>
   <223> Séquence du gène arsR d'Escherichia coli K12 souche MG1655
<400> 5
   atgcttgatt ataaagatga tgatgataaa ggctcatttc tgttacccat ccaattgttc        60
   aaaattcttg ctgatgaaac ccgtctgggc atcgttttac tgctcagcga actgggagag       120
   ttatgcgtct gcgatctctg cactgctctc gaccagtcgc agcccaagat ctcccgccac       180
   ctggcattgc tgcgtgaaag cgggctattg ctggaccgca agcaaggtaa gtgggttcat       240
   taccgcttat caccgcatat tccagcatgg gcggcgaaaa ttattgatga ggcctggcga       300
   tgtgaacagg aaaaggttca ggcgattgtc cgcaacctgg ctcgacaaaa ctgttccggg       360
   gacagtaaga acatttgcag ttaa                                              384
<210> 6
   <211> 61
   <212> DNA
   <213> Artificial Sequence
<220>
   <223> Amorce arsRR
<400> 6
   cttggatcct tagcctttat catcatcatc tttataatca ctgcaaatgt tcttactgtc        60
   c                                                                        61
<210> 7
   <211> 59
   <212> DNA
   <213> Artificial Sequence
<220>
   <223> Amorce arsRL
<400> 7
   ctagcatgct tgattataaa gatgatgatg ataaaggctc atttctgtta cccatccaa 59
<210> 8
   <211> 381
   <212> DNA
   <213> Artificial Sequence
<220>
   <223> séquence du gène arsR d'Escherichia coli K12 souche MG1655 dans lequel la séquence codante pour le peptide Flag a été insérée en position N-terminale, en aval du codon initiation ATG
<400> 8
   atggattata aagatgatga tgataaaggc tcatttctgt tacccatcca attgttcaaa        60
   attcttgctg atgaaacccg tctgggcatc gttttactgc tcagcgaact gggagagtta       120
   tgcgtctgcg atctctgcac tgctctcgac cagtcgcagc ccaagatctc ccgccacctg       180
   gcattgctgc gtgaaagcgg gctattgctg gaccgcaagc aaggtaagtg ggttcattac       240
   cgcttatcac cgcatattcc agcatgggcg gcgaaaatta ttgatgaggc ctggcgatgt       300
   gaacaggaaa aggttcaggc gattgtccgc aacctggctc gacaaaactg ttccggggac       360
   agtaagaaca tttgcagtta a                                                 381
<210> 9
   <211> 127
   <212> PRT
   <213> Artificial Sequence
<220>
   <223> Séquence de la protéine ArsRFlagN
<400> 9
   Met Leu Asp Tyr Lys Asp Asp Asp Asp Lys Gly Ser Phe Leu Leu Pro
   1               5                   10                  15
   Ile Gln Leu Phe Lys Ile Leu Ala Asp Glu Thr Arg Leu Gly Ile Val
               20                  25                  30
   Leu Leu Leu Ser Glu Leu Gly Glu Leu Cys Val Cys Asp Leu Cys Thr
           35                  40                  45
   Ala Leu Asp Gln Ser Gln Pro Lys Ile Ser Arg His Leu Ala Leu Leu
       50                  55                  60
   Arg Glu Ser Gly Leu Leu Leu Asp Arg Lys Gln Gly Lys Trp Val His
   65                  70                  75                  80
   Tyr Arg Leu Ser Pro His Ile Pro Ala Trp Ala Ala Lys Ile Ile Asp
                   85                  90                  95
   Glu Ala Trp Arg Cys Glu Gln Glu Lys Val Gln Ala Ile Val Arg Asn
               100                 105                 110
   Leu Ala Arg Gln Asn Cys Ser Gly Asp Ser Lys Asn Ile Cys Ser
           115                 120                 125
<210> 10
   <211> 9
   <212> PRT
   <213> Artificial Sequence
<220>
   <223> Peptide de reconnaissance : Flag
<400> 10
   Asp Tyr Lys Asp Asp Asp Asp Lys Gly
   1               5
<210> 11
   <211> 14
   <212> PRT
   <213> Artificial Sequence
<220>
   <223> Peptide de reconnaissance : peptide V5
<400> 11
   Gly Lys Pro Ile Pro Asn Pro Leu Leu Gly Leu Asp Ser Thr
   1               5                   10
<210> 12
   <211> 6
   <212> PRT
   <213> Artificial Sequence
<220>
   <223> Peptide de reconnaissance : His
<400> 12
   His His His His His His
   1               5
<210> 13
   <211> 10
   <212> PRT
   <213> Artificial Sequence
<220>
   <223> Peptide de reconnaissance : c-MYC
<400> 13
   Glu Gln Lys Leu Ile Ser Glu Glu Asp Leu
   1               5                   10
<210> 14
   <211> 9
   <212> PRT
   <213> Artificial Sequence
<220>
   <223> Peptide de reconnaissance : HA
<400> 14
   Tyr Pro Tyr Asp Val Pro Asp Tyr Ala
   1 5
<210> 15
   <211> 11
   <212> PRT
   <213> Artificial Sequence
<220>
   <223> Peptide de reconnaissance : VSV-G
<400> 15
   Tyr Thr Asp Ile Glu Met Asn Arg Leu Gly Lys
   1 5 10
<210> 16
   <211> 6
   <212> PRT
   <213> Artificial Sequence
<220>
   <223> Peptide de reconnaissance : AU5
<400> 16
   Thr Asp Phe Tyr Leu Lys
   1 5
<210> 17
   <211> 127
   <212> DNA
   <213> Artificial Sequence
<220>
   <223> E. coli R-factor R773 arsR gene (X16045)
<400> 17
   gaattccaag ttatctcacc taccttaagg taatagtgtg attaatcata tgcgtttttg 60
   gttatgtgtt gtttgactta atatcagagc cgagagatac ttgttttcta caaaggagag 120
   ggaaatg 127
<210> 18
   <211> 380
   <212> DNA
   <213> Artificial Sequence
<220>
   <223> ADNc codant pour ArsRFlagN optimisée
<400> 18
   ccatggacta caaagacgac gacgacaaag gtagctttct gctgccgatc cagctgttca 60
   aaattctggc agacgaaacc cgtctgggta ttgtgttact gctgagcgaa ttaggcgaac 120
   tgtgcgtttg cgatctgtgt accgcgttag atcagagtca gccgaaaatt agccgtcatc 180
   tggcgttatt acgcgaaagc ggtctgttac tggaccgtaa acagggcaaa tgggtccatt 240
   accgcttatc tccgcatatt ccggcttggg cagcaaaaat cattgacgaa gcctggcgtt 300
   gcgaacagga aaaagttcag gcgatcgtcc gtaacctggc acgtcaaaat tgtagcggcg 360
   atagcaaaaa catctcgagc 380
<210> 19
   <211> 24
   <212> DNA
   <213> Artificial Sequence
<220>
   <223> Séquence Tag
<400> 19
   gactacaaag acgacgacga caaa 24
<210> 20
   <211> 345
   <212> DNA
   <213> Artificial Sequence
<220>
   <223> séquence optimisée du gène arsR
<400> 20
   ggtagctttc tgctgccgat ccagctgttc aaaattctgg cagacgaaac ccgtctgggt 60
   attgtgttac tgctgagcga attaggcgaa ctgtgcgttt gcgatctgtg taccgcgtta 120
   gatcagagtc agccgaaaat tagccgtcat ctggcgttat tacgcgaaag cggtctgtta 180
   ctggaccgta aacagggcaa atgggtccat taccgcttat ctccgcatat tccggcttgg 240
   gcagcaaaaa tcattgacga agcctggcgt tgcgaacagg aaaaagttca ggcgatcgtc 300
   cgtaacctgg cacgtcaaaa ttgtagcggc gatagcaaaa acatc 345
<210> 21
   <211> 399
   <212> DNA
   <213> Artificial Sequence
<220>
   <223> ADNc de ArsRFlagN-6His
<400> 21
   atggactaca aagacgacga cgacaaaggt agctttctgc tgccgatcca gctgttcaaa        60
   attctggcag acgaaacccg tctgggtatt gtgttactgc tgagcgaatt aggcgaactg       120
   tgcgtttgcg atctgtgtac cgcgttagat cagagtcagc cgaaaattag ccgtcatctg       180
   gcgttattac gcgaaagcgg tctgttactg gaccgtaaac agggcaaatg ggtccattac       240
   cgcttatctc cgcatattcc ggcttgggca gcaaaaatca ttgacgaagc ctggcgttgc       300
   gaacaggaaa aagttcaggc gatcgtccgt aacctggcac gtcaaaattg tagcggcgat       360
   agcaaaaaca tctcgagcca ccaccaccac caccactga                              399
<210> 22
   <211> 132
   <212> PRT
   <213> Artificial Sequence
<220>
   <223> Protéine ArsRFlagN-6His
<400> 22
   Met Asp Tyr Lys Asp Asp Asp Asp Lys Gly Ser Phe Leu Leu Pro Ile
   1               5                   10                  15
   Gln Leu Phe Lys Ile Leu Ala Asp Glu Thr Arg Leu Gly Ile Val Leu
               20                  25                  30
   Leu Leu Ser Glu Leu Gly Glu Leu Cys Val Cys Asp Leu Cys Thr Ala
           35                  40                  45
   Leu Asp Gln Ser Gln Pro Lys Ile Ser Arg His Leu Ala Leu Leu Arg
       50                  55                  60
   Glu Ser Gly Leu Leu Leu Asp Arg Lys Gln Gly Lys Trp Val His Tyr
   65                  70                  75                  80
   Arg Leu Ser Pro His Ile Pro Ala Trp Ala Ala Lys Ile Ile Asp Glu
                   85                  90                  95
   Ala Trp Arg Cys Glu Gln Glu Lys Val Gln Ala Ile Val Arg Asn Leu
               100                 105                 110
   Ala Arg Gln Asn Cys Ser Gly Asp Ser Lys Asn Ile Ser Ser His His
           115                 120                 125
   His His His His
       130
<210> 23
   <211> 41
   <212> DNA
   <213> Artificial Sequence
<220>
   <223> Séquence fixant ArsR Pars1L biotinylé
<400> 23
   ctgcacttac acattcgtta agtcatatat gtttttgact t 41
<210> 24
   <211> 39
   <212> DNA
   <213> Artificial Sequence
<220>
   <223> Séquence fixant ArsR Pars1R
<400> 24
   aagtcaaaaa catatatgac ttaacgaatg tgtaagtgc 39
<210> 25
   <211> 41
   <212> DNA
   <213> Artificial Sequence
<220>
   <223> Séquence fixant ArsR Pars2L biotinylé
<400> 25
   ttttttttgc gcattcgtta agtcatatat gtttttgact t 41
<210> 26
   <211> 33
   <212> DNA
   <213> Artificial Sequence
<220>
   <223> Séquence fixant ArsR Pars2R
<400> 26
   aagtcaaaaa catatatgac ttaacgaatc ccc 33